(19) Europäisches Patentamt
European Patent Office
Office européen des brevets

(11) EP 4 159 209 A1

(12) EUROPEAN PATENT APPLICATION
published in accordance with Art. 153(4) EPC

(43) Date of publication:
**05.04.2023 Bulletin 2023/14**

(21) Application number: **21814638.9**

(22) Date of filing: **25.05.2021**

(51) International Patent Classification (IPC):
***A61K 31/095*** (2006.01) ***A61K 31/385*** (2006.01)
***A61P 27/02*** (2006.01) ***A61P 27/10*** (2006.01)
***C07C 323/12*** (2006.01) ***C07D 339/04*** (2006.01)

(52) Cooperative Patent Classification (CPC):
**A61K 31/095; A61K 31/385; A61P 27/02; A61P 27/10; C07C 323/12; C07D 339/04**

(86) International application number:
**PCT/JP2021/019808**

(87) International publication number:
**WO 2021/241582 (02.12.2021 Gazette 2021/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.05.2020 JP 2020091572**
**15.10.2020 JP 2020174232**

(71) Applicant: **Santen Pharmaceutical Co., Ltd.**
**Kita-ku**
**Osaka-shi**
**Osaka 530-8552 (JP)**

(72) Inventors:
- **KATO, Masatomo**
  **Ikoma-shi, Nara 630-0101 (JP)**
- **ODA, Tomoko**
  **Ikoma-shi, Nara 630-0101 (JP)**
- **HONDA, Takahiro**
  **Ikoma-shi, Nara 630-0101 (JP)**

(74) Representative: **Müller-Boré & Partner**
**Patentanwälte PartG mbB**
**Friedenheimer Brücke 21**
**80639 München (DE)**

# (54) SULFUR-CONTAINING COMPOUND USEFUL FOR TREATING OR PREVENTING PRESBYOPIA

(57) The present disclosure provides an agent for treating or preventing an eye disease such as presbyopia, comprising, as an active ingredient, a sulfur-containing compound having a specified structure.

EP 4 159 209 A1

## Description

Technical Field

The present invention relates to a sulfur-containing compound useful for the treatment or prevention of an eye disease such as presbyopia, and an agent for treating or preventing an eye disease such as presbyopia, which comprises the sulfur-containing compound as an active ingredient.

Background Art

Presbyopia is one of aging phenomena of the eye that begins around the age of 40 and is commonly called aged eyes. According to Non-Patent Document 1, presbyopia is defined as a disease state in which the accommodative amplitude decreases with aging (Age-Related Loss of Accommodation). In order to focus on something near or far away, it is necessary for the light that enters the eye to be refracted appropriately as it passes through the lens. Therefore, the eye has the function of adjusting the thickness of the lens such as contraction of the ciliary muscle located near the lens. The ocular tissues involved in the accommodation include lens, Zinn's zonule, lens capsule, and ciliary muscle. However, if the function of the ciliary muscle deteriorates due to aging, or if the lens elasticity (or, viscoelasticity) decreases, that is, the lens hardens, it becomes difficult to adjust the thickness of the lens, and it becomes difficult to focus on objects. This condition is presbyopia.

Patent Document 1 discloses experimental results in which lipoic acid can improve lens elasticity of a mouse to treat presbyopia, and also discloses synthesis examples of several lipoic acid derivatives (prodrugs). One of the disclosed prodrugs, lipoic acid choline ester (also known as EV06, UNR 844), is in clinical development in the United States as an eye drop.

Prior Art Document

Patent Document

Patent Document 1: WO 2010/147957

Non-Patent Document

Non-Patent Document 1: "Atarashii ganka" [A New Ophthalmology], Vol. 28, No. 7, 985-988, 2011

The disclosures of the prior art documents cited herein are hereby incorporated by reference in their entirety.

Summary

Technical Problem

An object of the present application is to provide a new measure for treating or preventing presbyopia, which is a very interesting challenge.

Solution to Problem

As a result of intensive research to solve the above problem, the present inventors have studied various sulfur-containing compounds for therapeutic effects on presbyopia, and have surprisingly found that a sulfur-containing compound having a specified structure more significantly improves lens elasticity as compared with the above-described lipoic acid choline ester that has been clinically developed, and thereby have reached the invention of the present disclosure. Specifically, the present disclosure provides the following aspects of the invention.

[1] An agent/composition for treating or preventing presbyopia, comprising, as an active ingredient, a compound represented by Formula [I]

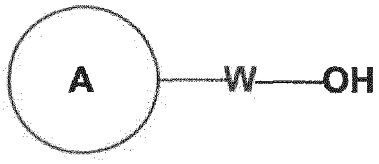 [I]

or a pharmaceutically acceptable salt thereof, wherein:

the A moiety is

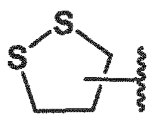 , 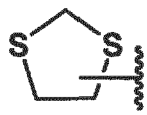 ,

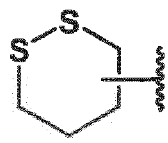 , 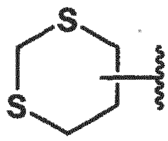 , 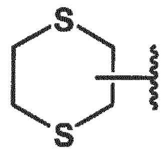

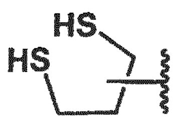 or 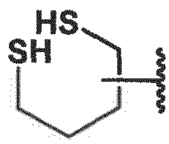 ,

wherein the A moiety may be optionally substituted with the same or different one to three (preferably one or two, even more preferably one) R1;

$R^1$, at each occurrence, is independently selected from the group consisting of -OH, halo, $C_{1-3}$ alkyl, and $C_{1-3}$ alkoxy;

W is $C_{1-10}$ alkylene (preferably $C_{3-8}$ alkylene, more preferably $C_{3-6}$ alkylene, still more preferably $C_{4-6}$ alkylene, still more preferably $C_5$ alkylene); in which one or two methylene groups in the alkylene may each be independently replaced with a divalent group selected from the group consisting of O, $NR^2$ in which $R^2$ is H or $C_{1-3}$ alkyl, and $S(O)_{0-2}$; and in which the alkylene may be optionally substituted with at least one (for example, one to five, one to three) substituent independently selected from the group consisting of -OH, halo, $C_{1-3}$ alkyl, and $C_{1-3}$ alkoxy.

[2] An agent/composition for treating or preventing an eye disease accompanied by a decrease in lens elasticity, comprising, as an active ingredient, a compound of Formula [I]

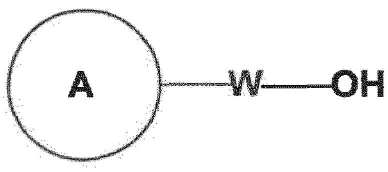 [I] ,

wherein the A moiety and W each has the same meaning as defined in [1],
or a pharmaceutically acceptable salt thereof.

[3] The agent/composition according to [2], wherein the eye disease is accompanied by a decrease in accommodative function of the eye.

[4] An agent/composition for treating or preventing an eye disease accompanied by a decrease in accommodative function of the eye, comprising, as an active ingredient, a compound of Formula [I]

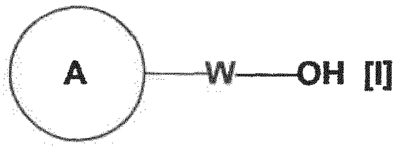
,

wherein the A moiety and W each has the same meaning as defined in [1],
or a pharmaceutically acceptable salt thereof.

[5] The agent/composition according to any one of [2] to [4], wherein the eye disease is presbyopia.

[6] A composition comprising a compound of Formula [I]

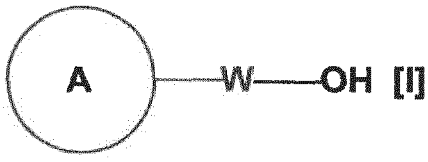
,

wherein the A moiety and W each has the same meaning as defined in [1],
or a pharmaceutically acceptable salt thereof,
wherein the composition has a high penetration property into lens.

[7] The agent/composition according to any one of [1] to [6], wherein the A moiety is

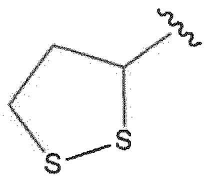 , 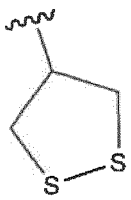 , 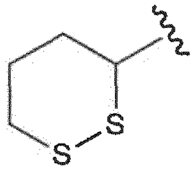 , 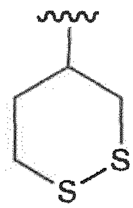 , 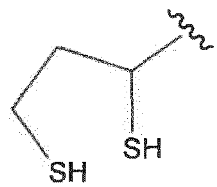 ,

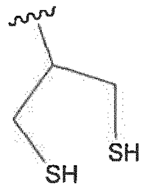 , 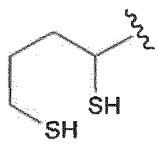 , or, 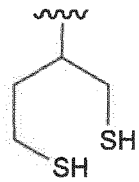 ,

wherein the A moiety may be optionally substituted with the same or different one to three (preferably one or two, even more preferably one) $R^1$.

[8] The agent/composition according to any one of [1] to [7], wherein the A moiety is

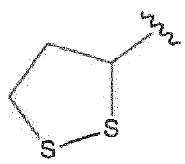 or 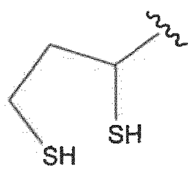 ,

wherein the A moiety may be optionally substituted with the same or different one to three (preferably one or two, even more preferably one) $R^1$,.

[9] The agent/composition according to any one of [1] to [8], wherein the A moiety is

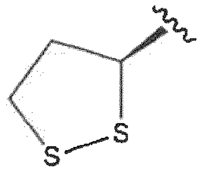

or

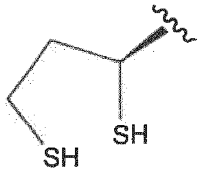

,

wherein the A moiety may be optionally substituted with the same or different one to three (preferably one or two, even more preferably one) $R^1$.

[10] The agent/composition according to any one of [1] to [9], wherein the A moiety is

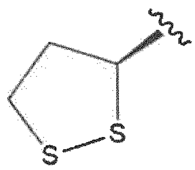

or

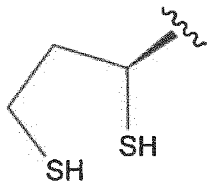

.

[11] The agent/composition according to any one of [1] to [10], wherein

W is alkylene which is $-(CH_2)_{1-10}-$ (preferably $-(CH_2)_{3-6}-$, more preferably $-(CH_2)_{4-6}-$, still more preferably $-(CH_2)_5-$), wherein the alkylene may be optionally substituted with at least one (for example, one to five, one to three) substituent independently selected from the group consisting of -OH, halo, $C_{1-3}$ alkyl, and $C_{1-3}$ alkoxy; or alternatively,
W is

- $(CH_2)_{1-5}$-O-$(CH_2)_{2-4}$- (preferably $-(CH_2)_{1-2}$-O-$(CH_2)_{2-3}$-, more preferably $-(CH_2)_2$-O-$(CH_2)_2$-),
- $(CH_2)_{1-2}$-O-$(CH_2)_{2-3}$-O-$(CH_2)_{2-3}$-,
- $(CH_2)_{1-5}$-S-$(CH_2)_{2-4}$- (preferably - $(CH_2)_{1-2}$-S-$(CH_2)_{2-3}$-),
- $(CH_2)_{1-2}$-S-$(CH_2)_{2-3}$-S-$(CH_2)_{2-3}$-,
- $(CH_2)_{1-5}$-NH-$(CH_2)_{2-4}$- (preferably - $(CH_2)_{1-2}$-NH-$(CH_2)_{2-3}$-), or
- $(CH_2)_{1-2}$-NH-$(CH_2)_{2-3}$-NH-$(CH_2)_{2-3}$-,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group), and the W may be optionally substituted with at least one (for example, one to five, one to three) substituent independently selected from the group consisting of -OH, halo, $C_{1-3}$ alkyl, and $C_{1-3}$ alkoxy.

[12] The agent/composition according to any one of [1] to [11], wherein W is alkylene which is $-(CH_2)_{1-10}-$(preferably $-(CH_2)_{3-6}-$, more preferably $-(CH_2)_{4-6}-$, still more preferably $-(CH_2)_5-$), wherein the alkylene may be optionally substituted with at least one (for example, one to five, one to three) substituent independently selected from the group consisting of -OH, halo, $C_{1-3}$ alkyl, and $C_{1-3}$ alkoxy.

[13] The agent/composition according to any one of [1] to [12], wherein W is alkylene which is $-(CH_2)_{1-10}-$(preferably $-(CH_2)_{3-6}-$, more preferably $-(CH_2)_{4-6}-$, still more preferably -(CH2)5-).

[14] The agent/composition according to any one of [1] to [11], wherein W is

- $(CH_2)_{1-5}$-O-$(CH_2)_{2-4}$- (preferably $-(CH_2)_{1-2}$-O-$(CH_2)_{2-3}$-, more preferably $-(CH_2)_2$-O-$(CH_2)_2$-),
- $(CH_2)_{1-2}$-O-$(CH_2)_{2-3}$-O-$(CH_2)_{2-3}$-,
- $(CH_2)_{1-5}$-S-$(CH_2)_{2-4}$- (preferably $-(CH_2)_{1-2}$-S-$(CH_2)_{2-3}$-),
- $(CH_2)_{1-2}$-S-$(CH_2)_{2-3}$-S-$(CH_2)_{2-3}$-,

- $(CH_2)_{1-5}$-NH-$(CH_2)_{2-4}$- (preferably -$(CH_2)_{1-2}$-NH-$(CH_2)_{2-3}$-), or
- $(CH_2)_{1-2}$-NH-$(CH_2)_{2-3}$-NH-$(CH_2)_{2-3}$-,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group), and the W may be optionally substituted with at least one (for example, one to five, one to three) substituent independently selected from the group consisting of -OH, halo, $C_{1-3}$ alkyl, and $C_{1-3}$ alkoxy.

[15] The agent/composition according to any one of [1] to [11] and [14], wherein W is

- $(CH_2)_{1-5}$-O-$(CH_2)_{2-4}$- (preferably -$(CH_2)_{1-2}$-O-$(CH_2)_{2-3}$-, more preferably -$(CH_2)_2$-O-$(CH_2)_2$-),
- $(CH_2)_{1-2}$-O-$(CH_2)_{2-3}$-O-$(CH_2)_{2-3}$-,
- $(CH_2)_{1-5}$-S-$(CH_2)_{2-4}$-(preferably -$(CH_2)_{1-2}$-S-$(CH_2)_{2-3}$-),
- $(CH_2)_{1-2}$-S-$(CH_2)_{2-3}$-S-$(CH_2)_{2-3}$-,
- $(CH_2)_{1-5}$-NH-$(CH_2)_{2-4}$-(preferably -$(CH_2)_{1-2}$-NH-$(CH_2)_{2-3}$-), or
- $(CH_2)_{1-2}$-NH-$(CH_2)_{2-3}$-NH-$(CH_2)_{2-3}$-,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group).

[16] The agent/composition according to any one of [1] to [15], comprising, as an active ingredient, a compound selected from the group consisting of:

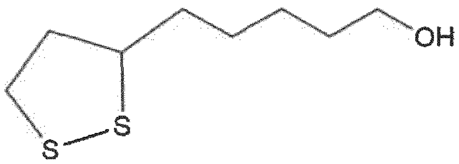
,
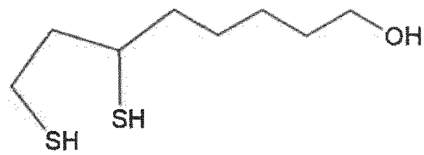
,
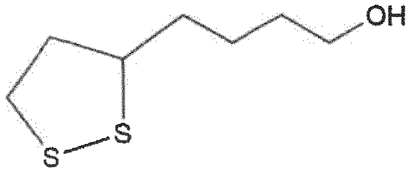
,
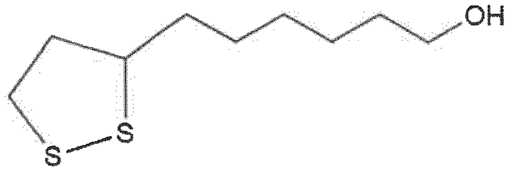
,

and

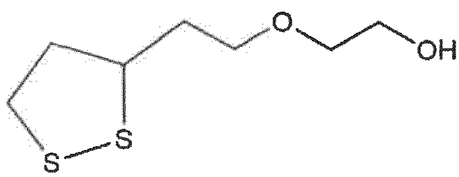
,

or a pharmaceutically acceptable salt thereof.

[17] The agent/composition according to any one of [1] to [16], comprising, as an active ingredient, a compound selected from the group consisting of:

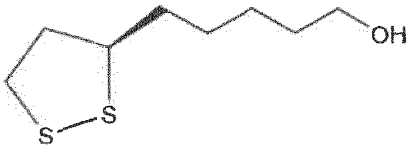 , 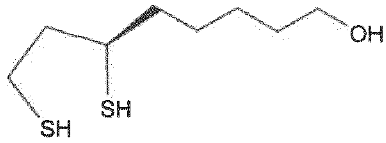 ,

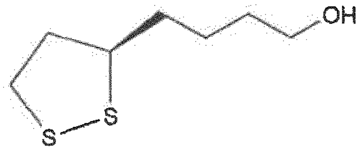 , 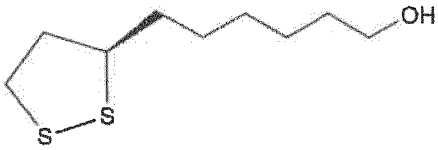 ,

and

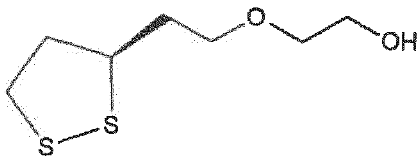 ,

or a pharmaceutically acceptable salt thereof.

[18] The agent/composition according to any one of [1] to [13] and [16], comprising, as an active ingredient, a compound:

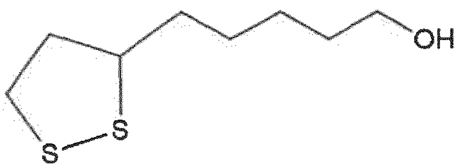 ,

or a pharmaceutically acceptable salt thereof.

[19] The agent/composition according to any one of [1] to [13] and [16] to [18], comprising, as an active ingredient, a compound:

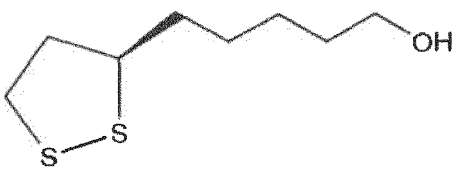 ,

or a pharmaceutically acceptable salt thereof.

[20] The agent/composition according to any one of [1] to [13] and [16], comprising, as an active ingredient, a compound:

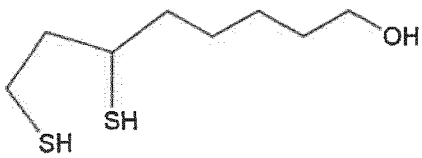 ,

or a pharmaceutically acceptable salt thereof.

[21] The agent/composition according to any one of [1] to [13], [16] to [17] and [20], comprising, as an active ingredient, a compound:

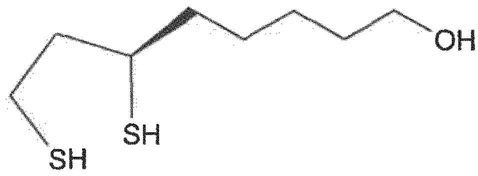

,

or a pharmaceutically acceptable salt thereof.

[22] The agent/composition according to any one of [1] to [13] and [16], comprising, as an active ingredient, a compound:

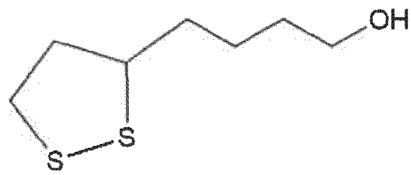

,

or a pharmaceutically acceptable salt thereof.

[23] The agent/composition according to any one of [1] to [13], [16] to [17] and [22], comprising, as an active ingredient, a compound:

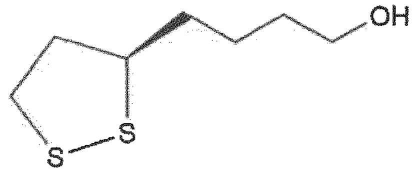

,

or a pharmaceutically acceptable salt thereof.

[24] The agent/composition according to any one of [1] to [13] and [16], comprising, as an active ingredient, a compound:

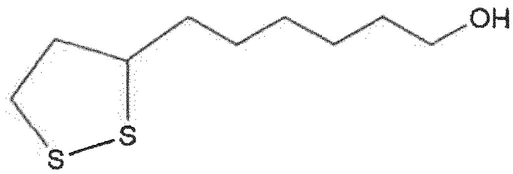

,

or a pharmaceutically acceptable salt thereof.

[25] The agent/composition according to any one of [1] to [13], [16] to [17] and [24], comprising, as an active ingredient, a compound:

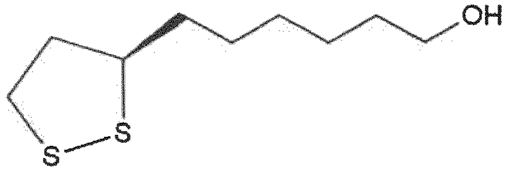

,

or a pharmaceutically acceptable salt thereof.

[26] The agent/composition according to any one of [1] to [11] and [14] to [16], comprising, as an active ingredient, a compound:

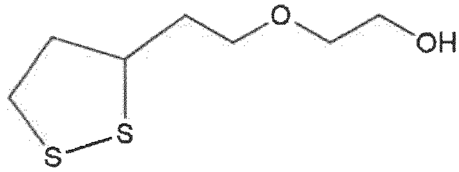

,

or a pharmaceutically acceptable salt thereof.

[27] The agent/composition according to any one of [1] to [11], [14] to [17] and [26], comprising, as an active ingredient, a compound:

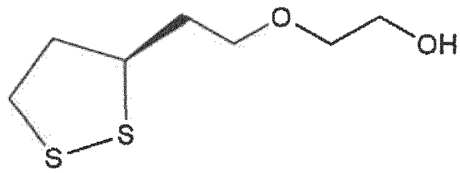

,

or a pharmaceutically acceptable salt thereof.

[28] The agent/composition according to any one of [1] to [27], wherein the agent/composition is for ophthalmic administration.

[29] The agent/composition according to any one of [1] to [28], wherein the agent/composition is an eye drop or an eye ointment.

[30] The agent/composition according to any one of [1] to [29], wherein the amount of the active ingredient comprised in the agent/composition is 0.00001 to 10% (w/v) (for example,, 0.001 to 3% (w/v), 0.003 to 2% (w/v), 0.01 to 2% (w/v), 0.03 to 1.5% (w/v), 0.03 to 0.5% (w/v), or 0.03 to 0.3% (w/v)).

[31] Use of a compound of Formula [I]

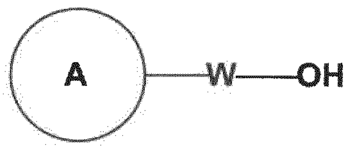 [I]

wherein the A moiety and W each has the same meaning as defined in [1]
or a pharmaceutically acceptable salt thereof, in the manufacture of an agent/composition for treating or preventing presbyopia, an eye disease accompanied by a decrease in lens elasticity, or an eye disease accompanied by a decrease in accommodative function of the eye.

[32] A compound of Formula [I]

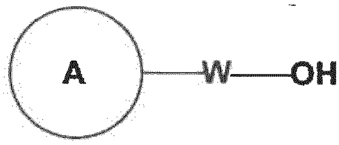 [I]

wherein the A moiety and W each has the same meaning as defined in [1] or a pharmaceutically acceptable salt thereof, for use in the treatment or prevention of presbyopia, an eye disease accompanied by a decrease in lens elasticity, or an eye disease accompanied by a decrease in accommodative function of the eye.

[33] A method for treating or preventing presbyopia, an eye disease accompanied by a decrease in lens elasticity, or an eye disease accompanied by a decrease in accommodative function of the eye, comprising administering to a subject in need thereof an effective amount of a compound of Formula [I]

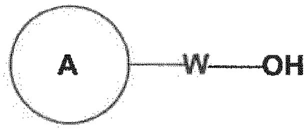 [I]

wherein the A moiety and W each has the same meaning as defined in [1] or a pharmaceutically acceptable salt thereof.

[34] A compound of Formula [I]

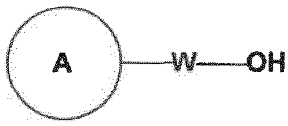 [I]

wherein the A moiety and W each has the same meaning as defined in [1], excluding the following compounds:

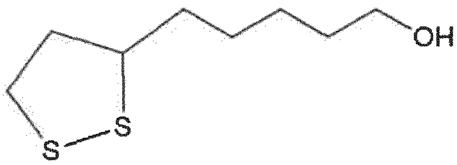 , and 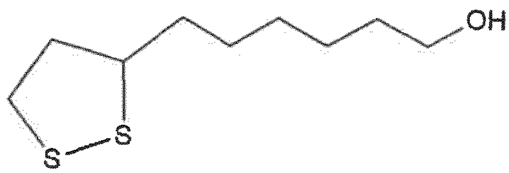 ,

or a pharmaceutically acceptable salt thereof.

[35] A compound selected from the group consisting of:

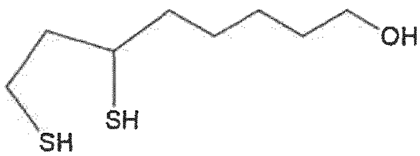 , 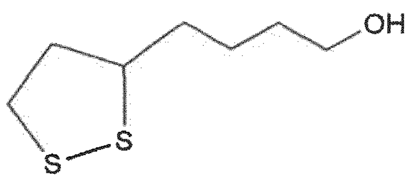 ,

and

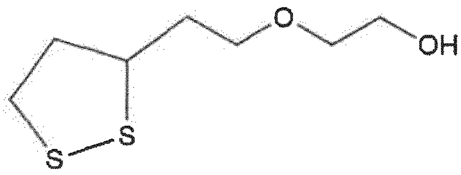

,

or a pharmaceutically acceptable salt thereof.

[36] A compound selected from the group consisting of:

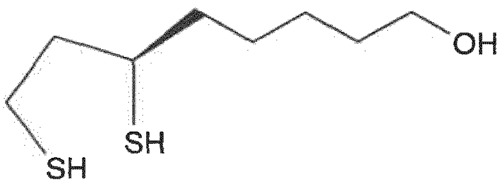

,

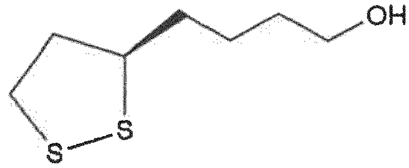

,

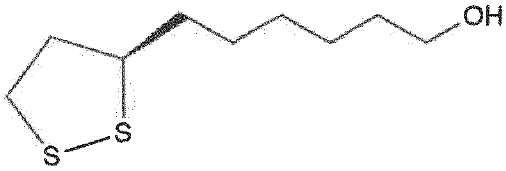

,

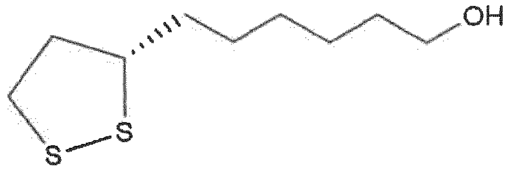

,

and

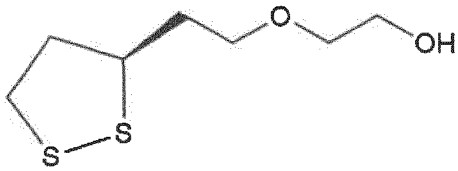

,

or a pharmaceutically acceptable salt thereof.

Each of the elements described in the above [1] to [36] may be optionally selected and combined.

Advantageous Effects of Invention

The compound of the present disclosure can improve the lens elasticity, which is important for lens thickness adjustment, and is therefore useful in the treatment or prevention of eye diseases such as presbyopia etc.

Description of Embodiments

Embodiments of the present invention are described in detail below.
The present disclosure provides a compound of Formula [I]

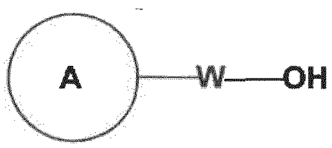

[I]

or a pharmaceutically acceptable salt thereof (hereinafter sometimes referred to as "the compound of the present invention"), wherein

the A moiety is

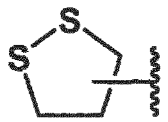 , 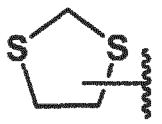 ,

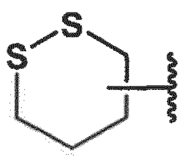 , 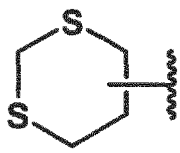 , 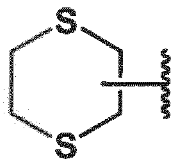

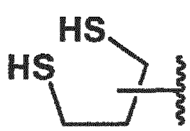 or 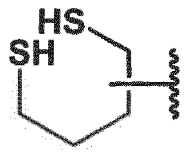 ,

wherein the A moiety may be optionally substituted with the same or different one to three (preferably one or two, even more preferably one) $R^1$;

$R^1$, at each occurrence, is independently selected from the group consisting of -OH, halo, $C_{1-3}$ alkyl, and $C_{1-3}$ alkoxy;

W is $C_{1-10}$ alkylene (preferably $C_{3-8}$ alkylene, more preferably $C_{3-6}$ alkylene, still more preferably $C_{4-6}$ alkylene, still more preferably $C_5$ alkylene); in which one or two methylene groups in the alkylene may each be independently replaced with a divalent group selected from the group consisting of O, $NR^2$ in which $R^2$ is H or $C_{1-3}$ alkyl, and $S(O)_{0-2}$; and in which the alkylene may be optionally substituted with at least one (for example, one to five, one to three) substituent independently selected from the group consisting of -OH, halo, $C_{1-3}$ alkyl, and $C_{1-3}$ alkoxy.

In one embodiment of the compound of the present invention, the A moiety is

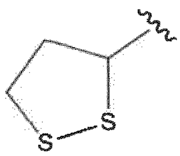 , 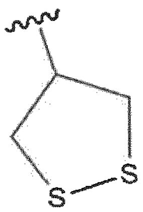 , 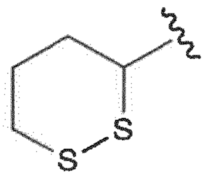 , 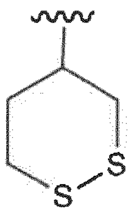 , 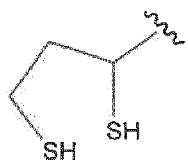 ,

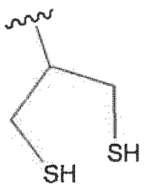 , 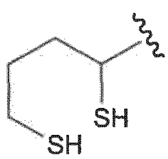 , or 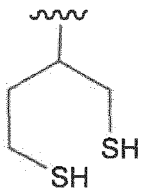 ,

wherein the A moiety may be optionally substituted with the same or different one to three (preferably one or two, even more preferably one) $R^1$.

In one embodiment of the compound of the present invention, the A moiety is

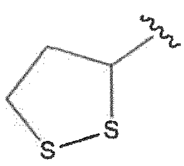 or 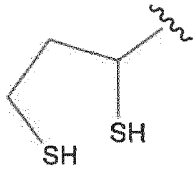 ,

wherein the A moiety may be optionally substituted with the same or different one to three (preferably one or two, even more preferably one) $R^1$.

In one embodiment of the compound of the present invention, the A moiety is

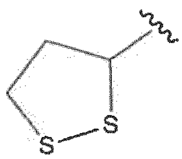 or 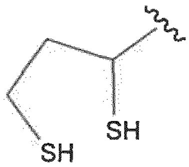 .

In one embodiment of the compound of the present invention, the A moiety is

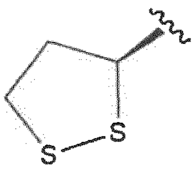 , 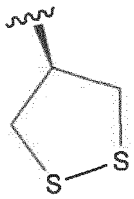 , 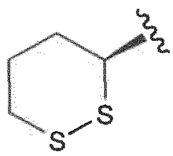 , 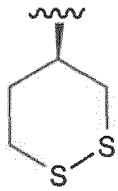 , 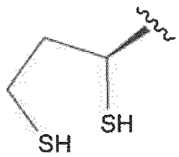 ,

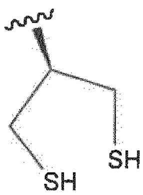 , 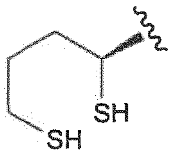 , or 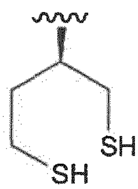 ,

wherein the A moiety may be optionally substituted with the same or different one to three (preferably one or two, even more preferably one) $R^1$.

In one embodiment of the compound of the present invention, the A moiety is

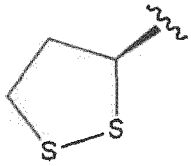 or 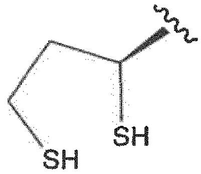 ,

wherein the A moiety may be optionally substituted with the same or different one to three (preferably one or two, even more preferably one) $R^1$.

In one embodiment of the compound of the present invention, the A moiety is

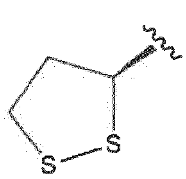 or 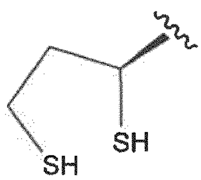 .

In one embodiment of the compound of the present invention, W is alkylene which is $-(CH_2)_{1-10}-$ (preferably $-(CH_2)_{3-6}-$, more preferably $-(CH_2)_{4-6}-$, still more preferably $-(CH_2)_5-$), wherein the alkylene may be optionally substituted with at least one (for example, one to five, one to three) substituent independently selected from the group consisting of -OH, halo, $C_{1-3}$ alkyl, and $C_{1-3}$ alkoxy; or alternatively,

W is

- $(CH_2)_{1-5}$-O-$(CH_2)_{2-4}$- (preferably $-(CH_2)_{1-2}$-O-$(CH_2)_{2-3}$-, more preferably $-(CH_2)_2$-O-$(CH_2)_2$-),
- $(CH_2)_{1-2}$-O-$(CH_2)_{2-3}$-O-$(CH_2)_{2-3}$-,
- $(CH_2)_{1-5}$-S-$(CH_2)_{2-4}$- (preferably $-(CH_2)_{1-2}$-S-$(CH_2)_{2-3}$-),
- $(CH_2)_{1-2}$-S-$(CH_2)_{2-3}$-S-$(CH_2)_{2-3}$-,
- $(CH_2)_{1-5}$-NH-$(CH_2)_{2-4}$- (preferably $-(CH_2)_{1-2}$-NH-$(CH_2)_{2-3}$-), or
- $(CH_2)_{1-2}$-NH-$(CH_2)_{2-3}$-NH-$(CH_2)_{2-3}$-,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group), and the W may be optionally substituted with at least one (for example, one to five, one to three) substituent independently selected from the group consisting of -OH, halo, $C_{1-3}$ alkyl, and $C_{1-3}$ alkoxy.

In one embodiment of the compound of the present invention, W is alkylene which is $-(CH_2)_{1-10}-$ (preferably $-(CH_2)_{3-6}-$, more preferably $-(CH_2)_{4-6}-$, still more preferably $-(CH_2)_5-$), wherein the alkylene may be optionally substituted with at least one (for example, one to five, one to three) substituent independently selected from the group consisting of -OH, halo, $C_{1-3}$ alkyl, and $C_{1-3}$ alkoxy; or alternatively,

W is $-(CH_2)_{1-5}$-O-$(CH_2)_{2-4}$- (preferably $-(CH_2)_{1-2}$-O-$(CH_2)_{2-3}$-, more preferably $-(CH_2)_2$-O-$(CH_2)_2$-), wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group), and the W may be optionally substituted with at least one (for example, one to five, one to three) substituent independently selected from the group consisting of -OH, halo, $C_{1-3}$ alkyl, and $C_{1-3}$ alkoxy.

In one embodiment of the compound of the present invention, W is alkylene which is $-(CH_2)_{1-10}-$ (preferably $-(CH_2)_{3-6}-$, more preferably $-(CH_2)_{4-6}-$, still more preferably $-(CH_2)_5-$); or

- $(CH_2)_{1-5}$-O-$(CH_2)_{2-4}$- (preferably $-(CH_2)_{1-2}$-O-$(CH_2)_{2-3}$-, more preferably $-(CH_2)_2$-O-$(CH_2)_2$-) (wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group)).

In one embodiment of the compound of the present invention, W is alkylene which is $-(CH_2)_{1-10}-$ (preferably $-(CH_2)_{3-6}-$, more preferably $-(CH_2)_{4-6}-$, still more preferably $-(CH_2)_5-$), wherein the alkylene may be optionally substituted with at least one (for example, one to five, one to three) substituent independently selected from the group consisting of -OH, halo, $C_{1-3}$ alkyl, and $C_{1-3}$ alkoxy.

In one embodiment of the compound of the present invention, W is alkylene which is $-(CH_2)_{1-10}-$ (preferably $-(CH_2)_{3-6}-$, more preferably $-(CH_2)_{4-6}-$, still more preferably $-(CH_2)_5-$).

In one embodiment of the compound of the present invention, W is

- $(CH_2)_{1-5}$-O-$(CH_2)_{2-4}$-(preferably $-(CH_2)_{1-2}$-O-$(CH_2)_{2-3}$-, more preferably $-(CH_2)_2$-O-$(CH_2)_2$-),
- $(CH_2)_{1-2}$-O-$(CH_2)_{2-3}$-O-$(CH_2)_{2-3}$-,
- $(CH_2)_{1-5}$-S-$(CH_2)_{2-4}$- (preferably $-(CH_2)_{1-2}$-S-$(CH_2)_{2-3}$-),
- $(CH_2)_{1-2}$-S-$(CH_2)_{2-3}$-S-$(CH_2)_{2-3}$-,
- $(CH_2)_{1-5}$-NH-$(CH_2)_{2-4}$- (preferably $-(CH_2)_{1-2}$-NH-$(CH_2)_{2-3}$-), or
- $(CH_2)_{1-2}$-NH-$(CH_2)_{2-3}$-NH-$(CH_2)_{2-3}$-,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached

to the oxygen atom of the hydroxy group), and the W may be optionally substituted with at least one (for example, one to five, one to three) substituent independently selected from the group consisting of -OH, halo, $C_{1-3}$ alkyl, and $C_{1-3}$ alkoxy.

In one embodiment of the compound of the present invention, W is $-(CH_2)_{1-5}-O-(CH_2)_{2-4}-$ (preferably $-(CH_2)_{1-2}-O-(CH_2)_{2-3}-$, more preferably $-(CH_2)_2-O-(CH_2)_2-$), wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group), and the W may be optionally substituted with at least one (for example, one to five, one to three) substituent independently selected from the group consisting of -OH, halo, $C_{1-3}$ alkyl, and $C_{1-3}$ alkoxy.

In one embodiment of the compound of the present invention, W is

- $(CH_2)_{1-5}-O-(CH_2)_{2-4}-$ (preferably $-(CH_2)_{1-2}-O-(CH_2)_{2-3}-$, more preferably $-(CH_2)_2-O-(CH_2)_2-$),
- $(CH_2)1-2$ $-O-(CH_2)_{2-3}-O-(CH_2)_{2-3}-$,
- $(CH_2)_{1-5}-S-(CH_2)_{2-4}-$ (preferably $-(CH_2)_{1-2}-S-(CH_2)_{2-3}-$),
- $(CH_2)_{1-2}-S-(CH_2)_{2-3}-S-(CH_2)_{2-3}-$,
- $(CH_2)_{1-5}-NH-(CH_2)_{2-4}-$ (preferably $-(CH_2)_{1-2}-NH-(CH_2)_{2-3}-$), or
- $(CH_2)_{1-2}-NH-(CH_2)_{2-3}-NH-(CH_2)_{2-3}-$,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group).

In one embodiment of the compound of the present invention, W is $-(CH_2)_{1-5}-O-(CH_2)_{2-4}-$ (preferably $-(CH_2)_{1-2}-O-(CH_2)_{2-3}-$, more preferably $-(CH_2)_2-O-(CH_2)_2-$), wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group).

In one embodiment of the compound of the present invention, W is

$-(CH_2)_{1-2}-O-(CH_2)_{2-3}-$,

$-(CH_2)_{1-2}-O-(CH_2)_{2-3}-O-(CH_2)_{2-3}-$,

$-(CH_2)_{1-2}-S-(CH_2)_{2-3}-$,

$-(CH_2)_{1-2}-S-(CH_2)_{2-3}-S-(CH_2)_{2-3}-$,

$-(CH_2)_{1-2}-NH-(CH_2)_{2-3}-$,

or

$-(CH_2)_{1-2}-NH-(CH_2)_{2-3}-NH-(CH_2)_{2-3}-$,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group), and the W may be optionally substituted with at least one (for example, one to five, one to three) substituent independently selected from the group consisting of -OH, halo, $C_{1-3}$ alkyl, and $C_{1-3}$ alkoxy.

In one embodiment of the compound of the present invention, W is

$-(CH_2)_{1-2}-O-(CH_2)_{2-3}-$,

$-(CH_2)_{1-2}-O-(CH_2)_{2-3}-O-(CH_2)_{2-3}-$,

$-(CH_2)_{1-2}-S-(CH_2)_{2-3}-$,

$-(CH_2)_{1-2}-S-(CH_2)_{2-3}-S-(CH_2)_{2-3}-$,

$-(CH_2)_{1-2}-NH-(CH_2)_{2-3}-$,

or

$-(CH_2)_{1-2}-NH-(CH_2)_{2-3}-NH-(CH_2)_{2-3}-$,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group).

In one embodiment of the compound of the present invention, W is

$-(CH_2)-O-(CH_2)_2-$,

$-(CH_2)-O-(CH_2)_3-$,

$-(CH_2)_2-O-(CH_2)_2-$,

$-(CH_2)_2-O-(CH_2)_3-$,

$-(CH_2)-O-(CH_2)_2-O-(CH_2)_2-$,

$-(CH_2)_2-O-(CH_2)_2-O-(CH_2)_2-$,

$-(CH_2)-O-(CH_2)_3-O-(CH_2)_3-$,

$-(CH_2)_2-O-(CH_2)_3-O-(CH_2)_3-$,

$-(CH_2)-S-(CH_2)_2-$,

$-(CH_2)-S-(CH_2)_3-$,

$-(CH_2)_2-S-(CH_2)_2-$,

$-(CH_2)_2-S-(CH_2)_{3-,}$

$-(CH_2)-S-(CH_2)_2-S-(CH_2)_2-$,

$-(CH_2)_2-S-(CH_2)_2-S-(CH_2)_2-$,

$-(CH_2)-S-(CH_2)_3-S-(CH_2)_3-$,

$-(CH_2)_2-S-(CH_2)_3-S-(CH_2)_3-$,

$-(CH_2)-NH-(CH_2)_2-$,

$-(CH_2)-NH-(CH_2)_3-$,

$-(CH_2)_2-NH-(CH_2)_2-$,

$-(CH_2)_2-NH-(CH_2)_3-$,

$-(CH_2)-NH-(CH_2)_2-NH-(CH_2)_2-$,

$-(CH_2)_2-NH-(CH_2)_2-NH-(CH_2)_2-$,

$-(CH_2)-NH-(CH_2)_3-NH-(CH_2)_3-$,

or

$-(CH_2)_2-NH-(CH_2)_3-NH-(CH_2)_3-$,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group), and the W may be optionally substituted with at least one (for example, one to five, one to three) substituent independently selected from the group consisting of -OH, halo, $C_{1-3}$ alkyl, and $C_{1-3}$ alkoxy.

In one embodiment of the compound of the present invention, W is

$-(CH_2)-O-(CH_2)_2-$,

$-(CH_2)-O-(CH_2)_3-$,

$-(CH_2)_2-O-(CH_2)_2-$,

$-(CH_2)_2-O-(CH_2)_3-$,

$-(CH_2)-O-(CH_2)_2-O-(CH_2)_2-$,

$-(CH_2)_2-O-(CH_2)_2-O-(CH_2)_2-$,

$-(CH_2)-O-(CH_2)_3-O-(CH_2)_3-$,

$-(CH_2)_2-O-(CH_2)_3-O-(CH_2)_3-$,

$-(CH_2)-S-(CH_2)_2-$,

$-(CH_2)-S-(CH_2)_3-$,

$-(CH_2)_2-S-(CH_2)_2-$,

$-(CH_2)_2-S-(CH_2)_3-$,

$-(CH_2)-S-(CH_2)_2-S-(CH_2)_2-$,

$-(CH_2)_2-S-(CH_2)_2-S-(CH_2)_2-$,

$-(CH_2)-S-(CH_2)_3-S-(CH_2)_3-$,

$-(CH_2)_2-S-(CH_2)_3-S-(CH_2)_3-$,

$-(CH_2)-NH-(CH_2)_2-$,

$-(CH_2)-NH-(CH_2)_3-$,

$-(CH_2)_2-NH-(CH_2)_2-$,

$-(CH_2)_2-NH-(CH_2)_3-$,

$-(CH_2)-NH-(CH_2)_2-NH-(CH_2)_2-$,

$-(CH_2)_2-NH-(CH_2)_2-NH-(CH_2)_2-$,

$-(CH_2)-NH-(CH_2)_3-NH-(CH_2)_3-$,

or

$-(CH_2)_2-NH-(CH_2)_3-NH-(CH_2)_3-$,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety (preferably, the leftmost bond is attached to the A moiety, and the rightmost bond is attached to the oxygen atom of the hydroxy group).

In one embodiment of the compound of the present invention, W is

$-(CH_2)-O-(CH_2)_2-$,

$-(CH_2)-O-(CH_2)_3-$,

$-(CH_2)_2-O-(CH_2)_2-$,

$-(CH_2)_2-O-(CH_2)_3-$,

$-(CH_2)-O-(CH_2)_2-O-(CH_2)_2-$,

$-(CH_2)_2-O-(CH_2)_2-O-(CH_2)_2-$,

$-(CH_2)-O-(CH_2)_3-O-(CH_2)_3-$,

$-(CH_2)_2-O-(CH_2)_3-O-(CH_2)_3-$,

$-(CH_2)-S-(CH_2)_2-$,

$-(CH_2)-S-(CH_2)_3-$,

$-(CH_2)_2-S-(CH_2)_2-$,

$-(CH_2)_2-S-(CH_2)_3-$,

$-(CH_2)-S-(CH_2)_2-S-(CH_2)_2-$,

$-(CH_2)_2-S-(CH_2)_2-S-(CH_2)_2-$,

$-(CH_2)-S-(CH_2)_3-S-(CH_2)_3-$,

$-(CH_2)_2-S-(CH_2)_3-S-(CH_2)_3-$,

$-(CH_2)-NH-(CH_2)_2-$,

$-(CH_2)-NH-(CH_2)_3-$,

$-(CH_2)_2-NH-(CH_2)_2-$,

$-(CH_2)_2-NH-(CH_2)_3-$,

$-(CH_2)-NH-(CH_2)_2-NH-(CH_2)_2-$,

$-(CH_2)_2-NH-(CH_2)_2-NH-(CH_2)_2-$,

$-(CH_2)-NH-(CH_2)_3-NH-(CH_2)_3-$,

or

$-(CH_2)_2-NH-(CH_2)_3-NH-(CH_2)_3-$,

wherein the leftmost bond is attached to the A moiety, and the rightmost bond is attached to the oxygen atom of the hydroxy group.

One embodiment of the compound of the present invention includes a compound or a pharmaceutically acceptable salt thereof, wherein said compound is selected from:

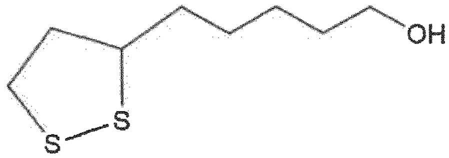
,
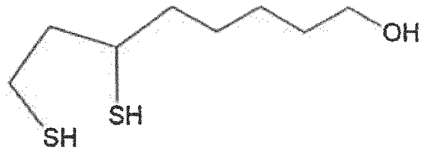
,
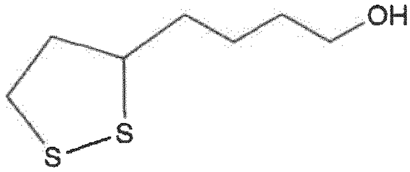
,
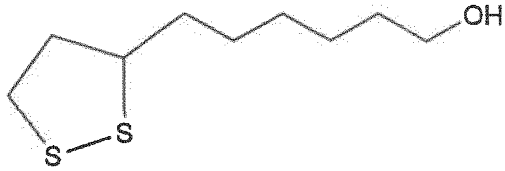
,

and

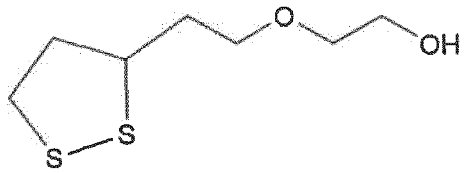
.

One embodiment of the compound of the present invention includes a compound or a pharmaceutically acceptable salt thereof, wherein said compound is selected from:

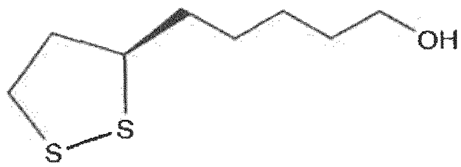
,
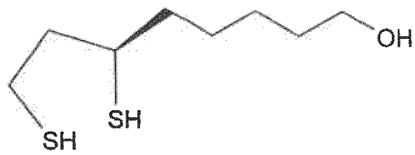
,
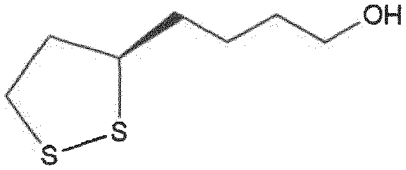
,
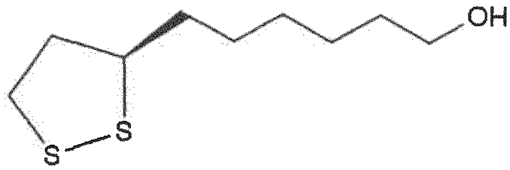
,
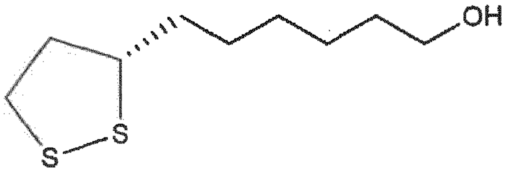
, and
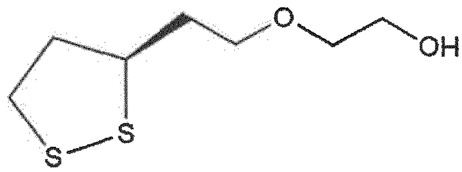
.

One embodiment of the compound of the present invention includes a compound of Formula [I] or a pharmaceutically acceptable salt thereof, wherein said compound of Formula [I] is not

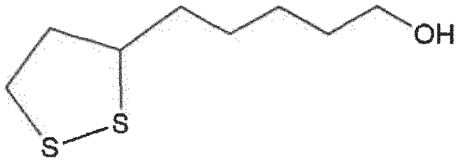

, or

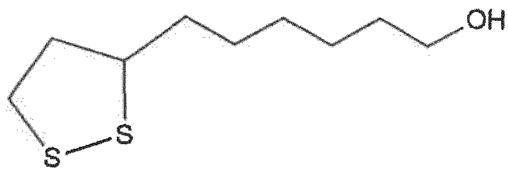

.

As described above, the compound of the present invention may be a dithiolane ring compound, a dithiane ring compound, or a pharmaceutically acceptable salt thereof. The 1,2-dithiolane ring compound and the 1,2-dithiane ring compound may each be interconverted with its ring-opened structure in vivo. Thus, the ring-opened structure, a compound having 1,3-propanedithiol moiety or 1,4-butanedithiol moiety, or a pharmaceutically acceptable salt thereof may have the same effect as the correcpoding 1,2-dithiolane or 1,2-dithiane ring compound.

The present disclosure further provides an agent/composition comprising, as an active ingredient, a compound of Formula [I] described above or a pharmaceutically acceptable salt thereof (hereinafter sometimes referred to as "the agent/composition of the present invention").

The agent/composition of the present invention may be an agent/composition comprising as an active ingredient one compound selected from the group consisting of the compounds of Formula [I] and pharmaceutically acceptable salts thereof described above, and may be an agent/composition comprising as an active ingredient more than one compound selected from the group consisting of the compounds of Formula [I] and pharmaceutically acceptable salts thereof described above.

Since 1, 2-dithiolane ring compound or a pharmaceutically acceptable salt thereof and the corresponding ring-opened compound having 1,3-propanedithiol moiety may interconvert in vivo, the agent/composition of the present invention may be an agent/composition comprising as an active ingredient one compound selected from them, and may be an agent/composition comprising as an active ingredient more than one compound selected from them.

Similarly, since 1,2-dithiane ring compound or a pharmaceutically acceptable salt thereof and the corresponding ring-opened compound having 1,4-butanedithiol moiety may interconvert in vivo, the agent/composition of the present invention may be an agent/composition comprising as an active ingredient one compound selected from them, and may be an agent/composition comprising as an active ingredient more than one compound selected from them.

The compound/agent/composition of the present invention may be used for treating or preventing presbyopia. The compound/agent/composition of the present invention may be used to improve lens elasticity. In addition, the compound/agent/composition of the present invention may be used to improve eye accommodation. Further, the compound of the present invention may efficiently penetrate to the lens compared to lipoic acid, and may be used for a composition having a high penetration property into lens.

Since the compound of the present invention is an alcohol compound having an OH group as shown in formula (I), it may have a characteristic that it does not undergo hydrolysis in vivo as the corresponding ester compound, etc.

In one embodiment of the compound of the present invention, examples of the A moiety include the following groups, each of which may be substituted. In the following formulae, the bond traversing the ring means that "-W-OH" may be located at any of the available positions in the ring.

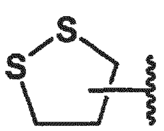

,

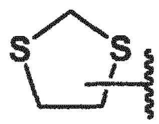

,

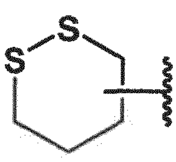

,

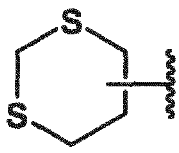

or

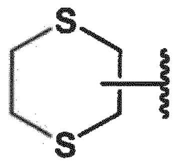

;

In one embodiment of the compound of the present invention, examples of the A moiety include the following groups, each of which may be substituted. In the following formulae, the bond traversing other bond means that "-W-OH" may be located at any of the available positions in the group. In one embodiment of the compound of the present invention, "-W-OH" binds to a carbon atom.

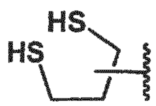

or

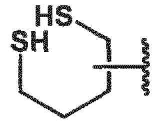

In the present disclosure, "halo" means fluoro, chloro, bromo or iodo.

In the present disclosure, "$C_{1-3}$ alkoxy" means a straight or branched-chain alkoxy having one to three carbon atoms. Examples of "$C_{1-3}$ alkoxy" include methoxy, ethoxy, propoxy, isopropoxy.

In the present disclosure, "$C_{1-3}$ alkyl" means a straight or branched-chain saturated hydrocarbon group having one to three carbon atoms. Examples of "$C_{1-3}$ alkyl" include methyl, ethyl, propyl, isopropyl.

In the present disclosure, "$C_{1-10}$ alkylene" means a divalent straight-chain saturated hydrocarbon group having one to ten carbon atoms, in which one or two methylene groups in the alkylene may each be independently replaced with a divalent group selected from the group consisting of O, $NR^2$ in which $R^2$ is H or $C_{1-3}$ alkyl, and $S(O)_{0-2}$.

Examples of the alkylene include methylene, dimethylene, trimethylene, tetramethylene, pentamethylene, hexamethylene, heptamethylene, octamethylene, nonamethylene, and decamethylene.

Other examples of the alkylene include:

$-(CH_2)-O-(CH_2)_2-$,

$-(CH_2)-O-(CH_2)_3-$,

$-(CH_2)_2-O-(CH_2)_2-$,

$-(CH_2)_2-O-(CH_2)_3-$,

$-(CH_2)-O-(CH_2)_2-O-(CH_2)_2-$,

$-(CH_2)_2-O-(CH_2)_2-O-(CH_2)_2-$,

$-(CH_2)-O-(CH_2)_3-O-(CH_2)_3-$,

$-(CH_2)_2-O-(CH_2)_3-O-(CH_2)_3-$,

$-(CH_2)-S-(CH_2)_2-$,

$-(CH_2)-S-(CH_2)_3-$,

$-(CH_2)_2-S-(CH_2)_2-$,

$-(CH_2)_2-S-(CH_2)_3-$,

$-(CH_2)-S-(CH_2)_2-S-(CH_2)_2-$,

$-(CH_2)_2-S-(CH_2)_2-S-(CH_2)_2-$,

$-(CH_2)-S-(CH_2)_3-S-(CH_2)_3-$,

$-(CH_2)_2-S-(CH_2)_3-S-(CH_2)_3-$,

$-(CH_2)-NH-(CH_2)_2-$,

$-(CH_2)-NH-(CH_2)_3-$,

$-(CH_2)_2-NH-(CH_2)_2-$,

$-(CH_2)_2-NH-(CH_2)_3-$,

$-(CH_2)-NH-(CH_2)_2-NH-(CH_2)_2-$,

$-(CH_2)_2-NH-(CH_2)_2-NH-(CH_2)_2-$,

$-(CH_2)-NH-(CH_2)_3-NH-(CH_2)_3-$,

and

$-(CH_2)_2-NH-(CH_2)_3-NH-(CH_2)_3-$,

in each of which, in Formula [I], the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety. Preferably, the leftmost bond is attached to the A moiety, and the rightmost bond is attached to the oxygen atom of the hydroxy group.

In one embodiment of the compound of the present invention, pharmaceutically acceptable salts are not particularly limited as long as they are pharmaceutically acceptable salts. Examples of pharmaceutically acceptable salts include, inorganic salts such as hydrochlorides, hydrobromides, hydroiodides, nitrates, sulfates, phosphates, etc.; organic acid salts such as acetates, trifluoroacetates, benzoates, oxalates, malonates, succinates, maleates, fumarates, tartrates, citrates, methanesulfonates, ethanesulfonates, trifluoromethanesulfonates, benzenesulfonates, *p*-toluenesulfonates, glutamates, aspartates, etc.; metal salts such as sodium salts, potassium salts, calcium salts, and magnesium salts, etc.; inorganic salts such as ammonium salts, etc.; and organic amine salts such as triethylamine salts, guanidine salts, etc. Examples of pharmaceutically acceptable salts include preferably sodium salts and potassium salts.

In one embodiment of the compound of the present invention, the compound of the present invention may be in the form of hydrates or solvates.

The agent/composition of the present invention may comprise a single type or a plurality of types of the compound of the present invention.

A compound of Formula [I] or a pharmaceutically acceptable salt thereof may have isomers, such as tautomers, geometric isomers or optical isomers, but formulae, chemical structures or chemical names without specifying a stereochemistry herein may include all isomers which may exist and a mixture in any ratio of the isomers, unless otherwise specified.

The amount of the compound of the present invention comprised in the agent/composition of the present invention (the total amount when a plurality of the compounds of the present invention is comprised) is not particularly limited and may be selected from a wide range depending on dosage forms etc.

For example, the amount of the compound of the present invention comprised in the agent/composition of the present invention is 0.00001 to 10% (w/v), preferably 0.0001 to 5% (w/v), more preferably 0.001 to 3% (w/v), even more preferably 0.003 to 2% (w/v), even more preferably 0.01 to 2% (w/v), particularly preferably 0.03 to 1.5% (w/v), more preferably 0.03 to 0.5% (w/v), and even more preferably 0.03 to 0.3% (w/v). An example of the lower limit of the amount is 0.00001% (w/v), a preferable example is 0.0001% (w/v), a more preferable example 0.001% (w/v), a more preferable example is 0.003% (w/v), a particularly preferable example is 0.01% (w/v), a further particularly preferable example is 0.02% (w/v), and a further particularly preferable example is 0.03% (w/v). An example of the upper limit of the amount is 10% (w/v), a preferable example is 5% (w/v), a more preferable example is 3% (w/v), a particularly preferable example is 2% (w/v), a further particularly preferable example is 1.5% (w/v), and a further particularly preferable example is 1% (w/v). In particular, from the viewpoint of suppressing eye irritation, the upper limit of the amount is preferably 1.5% (w/v), more preferably 0.3% (w/v), even more preferably 0.1% (w/v). A preferred range of the amount may be indicated by a combination of the above examples of lower and upper limits.

In addition, for example, the amount of the compound of the present invention comprised in the agent/composition of the present invention is 0.00001 to 10% (w/w), preferably 0.0001 to 5% (w/w), more preferably 0.001 to 3% (w/w), even more preferably 0.003 to 2% (w/w), even more preferably 0.01 to 2% (w/w), particularly preferably 0.03 to 1.5% (w/w), more preferably 0.03 to 0.5% (w/w), and even more preferably 0.03 to 0.3% (w/w). An example of the lower limit of the amount is 0.00001% (w/w), a preferable example is 0.0001% (w/w), a more preferable example is 0.001% (w/w), a even more preferable example is 0.0003% (w/w), a particularly preferable example is 0.01% (w/w), a further particularly preferable example is 0.02% (w/w), and a further particularly preferable example is 0.03% (w/w). An example of the upper limit of the amount is 10% (w/w), a preferable example is 5% (w/w), a more preferable example is 3% (w/w), a particularly preferable example is 2% (w/w), a further particularly preferable example is 1.5% (w/w), and a further particularly preferable example is 1% (w/w). In particular, from the viewpoint of suppressing eye irritation, the upper limit of the amount is preferably 1.5% (w/w), more preferably 0.3% (w/w), even more preferably 0.1% (w/w). A preferred range of the amount may be indicated by a combination of the above examples of lower and upper limits.

In the present disclosure, "% (w/v) " means the mass (g) of the active ingredient (the compound of the present invention) or an additive (surfactant, etc.) comprised in 100 mL of an agent. For example, "0.01% (w/v) of the compound

of the present invention" means that the amount of the compound of the present invention comprised in 100 mL of an agent is 0.01g.

In the present disclosure, "% (w/w)" means the mass (g) of the active ingredient (the compound of the present invention) or an additive (surfactant, etc.) comprised in 100 g of an agent. For example, "0.01% (w/w) of the compound of the present invention" means that the amount of the compound of the present invention comprised in 100 g of an agent is 0.01g.

When the compound of the present invention is in the form of salt, or in the form of hydrate or solvate (including the form of hydrate or solvate of the salt), the amount of the compound of the present invention comprised in an agent may mean the mass of the salt, hydrate, or solvate (including the hydrate or solvate of the salt) added into the agent, or may mean the mass converted as the free form, preferably may mean the mass converted as the free form.

In this disclosure, the term "presbyopia" means a symptom/disease that is determined to be presbyopia based on general criteria used by a physician or professional.

For example, diagnostic criteria for presbyopia include:

> Decreased near vision is noticed as a subjective symptom in a binocular vision test, and a binocular daily life visual acuity, which is a binocular distant visual acuity measured under the same condition as daily life, is less than 0.4 at 40 cm distance(clinical presbyopia); and / or
> With or without subjective symptoms, under unilateral best-correction where a corrected visual acuity of one eye is equal to or more than 1.0 (decimal visual acuity), accommodative amplitude is less than 2.5 Diopters" (medical presbyopia).

However, if an accommodometer etc. is not available, a simple criterion wherein a visual acuity at 40 cm is less than 0.4 may be used.

In the present disclosure, the term "an eye disease accompanied by a decrease in lens elasticity" refers to an eye disease considered in the field of ophthalmology to be accompanied by a decrease in lens elasticity, including, for example, presbyopia (e.g., presbyopia due to aging), and a hardening of the lens induced by drugs and the like.

In the present disclosure, the term "accommodative function of the eye" refers to an eye function that automatically focuses on distant and/or near objects. The term "an eye disease accompanied by a decrease in accommodative function of the eye" refers to an eye disease considered in the field of ophthalmology to be accompanied by a decrease in accommodative function of the eye, including, for example, presbyopia (e.g., presbyopia due to aging), and a hardening of the lens induced by drugs etc., and decreased accommodation function induced by seeing near objects for a long time.

The efficacy of the agent/composition of the present invention may be evaluated, for example, as an increase in "accommodative amplitude of the eye".

The accommodative amplitude of the eye can be measured as a Diopter (D) which can be determined by the following expression 1:

$$\text{Diopter (D)} = 1/\text{Near Point Distance (m)} \quad \text{(Expression 1)}.$$

In general, the accommodative amplitude of the eye is greater than 10 diopters at 10 years, then gradually decreases to about 3 diopters at about 45 years and is almost lost at about 60 years. When the accommodative amplitude decreases to about 3 diopters, it becomes difficult to focus on near objects (about 30 cm) in daily life, and subjective symptoms of presbyopia appear.

The efficacy of the agent/composition of the present invention may be evaluated, for example, as an improvement in "visual acuity". The visual acuity can be measured as near visual acuity (uncorrected visual acuity, distance-corrected near visual acuity, corrected visual acuity) and can be measured by using decimal visual acuity, fractional visual acuity, or logMAR.

In general, when near visual acuity which is defined to be measured at about 40 cm decreases to below 0.4, it causes difficulty in seeing near objects, and subjective symptoms of presbyopia appear. The agent/composition of the present invention may be used to improve near visual acuity (e.g., distance-corrected near visual acuity).

The agent/composition of the present invention may begin to exhibit an efficacy within one year, preferably within six months, more preferably within one month, more preferably within one week, and even more preferably within one day after the administration. Further, once an efficacy is exerted, the efficacy may be exerted continuously until after one day, preferably until after one week, more preferably until after one month, more preferably until after six months, particularly preferably until after one year, and even more preferably until after three years.

The agent/composition of the present invention may be administered, for example, so as to increase the accommodative amplitude of the eye by at least about 0.5 diopters (preferably at least about 1 diopter, more preferably at least about 1.5 diopters, more preferably at least about 2 diopters, even more preferably at least about 3 diopters, and

still more preferably at least about 4 diopters, particularly preferably at least about 5 diopters, and still more preferably at least about 10 diopters).

The agent/composition of the present invention may be administered, for example, so as to increase distance-corrected near visual acuity (DCNVA) by at least about 0.5 logMAR (preferably about at least 1.0 logMAR, more preferably about at least 1.5 logMAR, even more preferably about 2.0 logMAR, even more preferably about 3.0 logMAR, particularly preferably about 4.0 logMAR, particularly preferably about 5.0 logMAR, and even more preferably about 6.0 logMAR).

The term "distance-corrected near visual acuity" generally refers to near visual acuity measured with distance visual acuity corrected to ≤ 0.0 logMAR (decimal visual acuity of 1.0 or more).

The agent/composition of the present invention may be administered, for example, so as to restore the accommodative amplitude of the eye to at least about 0.5 diopters (preferably at least about 1 diopter, more preferably at least about 1.5 diopters, more preferably at least about 2 diopters, more preferably at least about 3 diopters, particularly preferably at least about 4 diopters, particularly preferably at least about 5 diopters, and still more preferably at least about 10 diopters).

The agent/composition of the present invention may be administered, for example, so as to restore the distance-corrected near visual acuity (DCNVA) to at least about 0.5 logMAR (preferably at least about 1.0 logMAR, more preferably at least about 1.5 logMAR, even more preferably about 2.0 logMAR, even more preferably about 3.0 logMAR, particularly preferably about 4.0 logMAR, particularly preferably about 5.0 logMAR, and even more preferably about 6.0 logMAR).

In the present disclosure, the treatment or prevention of presbyopia includes increasing an elasticity of the lens, improving an ability to adjust a thickness of lens, and/or improving an accommodative function of the eye.

Although subjective symptoms of presbyopia generally appear at about 45 years of age as mentioned above, age-related decline in eye accommodation has been progressing since teens. The agent/composition of the present invention may be used after the subjective symptoms of presbyopia appear, and may be used to prevent and/or delay progression of presbyopia before the subjective symptoms of presbyopia appear.

In the present disclosure, "A high penetration property into lens" means that a compound is more delivered to the lens when administered (preferably by eye drop administration) into the body as compared with lipoic acid. The method for testing whether or not "a high penetration property into lens" is shown is not particularly limited, but, for example, it can be assessed by measuring the concentration of lipoic acid in the lens, aqueous humor, etc. extracted after administering the compound into the body using a high performance liquid chromatography tandem mass spectrometer (LC-MS/MS).

The subjects of administration of the agent/composition of the present invention are mammals including livestock such as cattle and pigs, rabbits, monkeys, dogs, cats, and humans, preferably humans.

In this disclosure, "treatment (treating)" and "prevention (preventing)" may include, in addition to treating and preventing a disease, alleviating symptoms of the disease, delaying progression of the disease, suppressing symptoms of the disease, and inducing improvement in symptoms of the disease.

The agent/composition of the present invention may be administered orally or parenterally (e.g., ocularly, nasally, transdermally, transmucosally, by injection, etc.). From the viewpoint that the agent/composition of the present invention may be less irritating to the eye and exerts superior effects, the agent/composition of the present invention is preferably administered into eye. The agent/composition of the present invention may be prepared in the usual manner in the art by mixing the active ingredient with, for example, one or more pharmaceutically acceptable additives, for example, in the form of oral preparations such as tablets, capsules, granules, powders, lozenges, syrups, emulsions, suspensions, and the like, or parenteral preparations such as eye drops, ophthalmic ointments, injections, suppositories, nasal preparations, and the like. Preferred formulations of the agent/composition of the present invention include eye drops and eye ointments.

Pharmaceutically acceptable additives that may be comprised in the agent/composition of the present invention are not particularly limited and may be selected as appropriate according to the route of administration, formulation, etc. Examples of such pharmaceutically acceptable additives include, for example, surfactants, buffers, tonicity agents, stabilizers, preservatives, antioxidants, thickeners, solubilizing agents, suspending agents, bases, solvents, pH adjusters, excipients, disintegrating agents, binders, fluidizers, lubricants, preservatives, antioxidants, coloring agents, sweetening agents, and the like.

When the agent/composition of the present invention is an eye drop, examples of additives that may be used include surfactants, buffers, tonicity agents, stabilizers, preservatives, antioxidants, thickeners, solvents, pH adjusters, and the like.

Examples of surfactants include cationic surfactants, anionic surfactants, nonionic surfactants and the like.

When a surfactant is added to the agent/composition of the present invention, the amount of the surfactant comprised in the agent may be appropriately adjusted depending on the type of the surfactant, etc., and is preferably, for example, 0.01 to 1% (w/v).

Examples of buffers include phosphoric acid or salts thereof, which may be hydrates or solvates thereof.

Examples of the phosphoric acid or salts thereof include phosphoric acid, trisodium phosphate, sodium dihydrogenphosphate, sodium hydrogen phosphate (disodium hydrogenphosphate) and the like, which may be hydrates thereof.

When a buffer is added to the agent/composition of the present invention, the amount of the buffer comprised in the agent may be appropriately adjusted depending on the type of the buffer, etc., but for example, 0.001 to 10% (w/v) is preferable, and 0.01 to 5% (w/v) is more preferable. Two or more kinds of buffers may be used together.

Examples of tonicity agents include ionic tonicity agents and nonionic tonicity agents. Examples of the ionic tonicity agents include sodium chloride and the like.

When a tonicity agent is added to the agent/composition of the present invention, the amount of the tonicity agent comprised in the agent may be appropriately adjusted according to the type of the tonicity agent or the like, but for example, 0.001 to 10% (w/v) is preferable, and 0.01% to 5% (w/v) is more preferable.

Examples of thickeners include hydroxypropyl methylcellulose and the like.

When a thickener is added to the agent/composition of the present invention, the amount of the thickener may be appropriately adjusted according to the type of the thickener or the like, but for example, 0.001 to 5% (w/v) is preferable, and 0.01% to 3% (w/v) is more preferable.

When the agent/composition of the present invention is an aqueous formulation (e.g., eye drops), the pH is preferably 4 to 8 and more preferably 5 to 7.

Examples of solvents include water, physiological saline and the like.

Examples of the agent/composition of the present invention which is an aqueous preparation (e.g., eye drop) include aqueous preparations comprising the compound of the present invention, water, and an additive selected from ethyl pyruvate, sodium dihydrogenphosphate monohydrate ($NaH_2PO_4.H_2O$), disodium hydrogenphosphate ($Na_2HPO_4$), hydroxypropyl methylcellulose, NaCl, polyoxyl 35 castor oil, benzyl benzoate, and a mixture thereof. Here, said "a mixture thereof" means any combination of the listed specific additives.

As used herein, the term "an effective amount" is the amount of the active ingredient required to provide a patient benefit in the symptoms of a disease.

A dosage and administration of the agent/composition of the present invention is not particularly limited as long as the dosage and administration are sufficient to achieve the desired medicinal effect, and may be appropriately selected according to the symptoms of the disease, the age and weight of the patient, the dosage form of the agent, etc.

For example, in the case of eye drops, a single dose of 1 to 5 drops (preferably 1 to 3 drops, more preferably 1 to 2 drops, particularly preferably 1 drop) may be instilled 1 to 4 times per day (preferably 1 to 3 times per day, more preferably 1 to 2 times per day, particularly preferably once per day), every day or at an interval of from one day to one week. The "one drop" is usually about 0.01 to about 0.1 mL, preferably about 0.015 to about 0.07 mL, more preferably about 0.02 to about 0.05 mL, and particularly preferably about 0.03 mL.

In one embodiment, the agent of the present invention have an immediate effect on presbyopia, an eye disease accompanied by a decrease in lens elasticity, or an eye disease accompanied by a decrease in accommodative function of the eye, for example, compared to EV06 or other lipoic acid prodrug(s).

The duration of administration of the agent of the present invention may be determined by a physician or professional.

In one embodiment, the agent of the present invention may be an ophthalmic administration agent such as an eye drop (e.g., solution, emulsion, suspension) and an eye ointment, and may be used continuously for at least 2 days, at least 3 days, at least 7 days, at least 10 days.

In one embodiment, the agent of the present invention may be administered at least once (e.g., at least twice, at least three times) a day.

In one embodiment, the agent of the present invention, when administered to the eye, may be less irritating to the eye while having an effect on presbyopia, an eye disease accompanied by a decrease in lens elasticity, or an eye disease accompanied by a decrease in accommodative function of the eye.

## Synthesis

The compounds of the present invention can be prepared by a number of methods well known to those skilled in the art of organic synthesis. The compound of the present invention may be synthesized using methods of the following examples, or variations thereof well recognized by those skilled in the art, in conjunction with synthetic methods known in the art of organic synthetic chemistry. Preferred methods include, but are not limited to, those described in the following examples.

## Examples

The synthesis of compounds and the results of pharmacological, pharmacokinetic, and safety studies are shown below for a better understanding of the present invention and are not intended to limit the scope of the present invention.

[Synthesis of Compound]

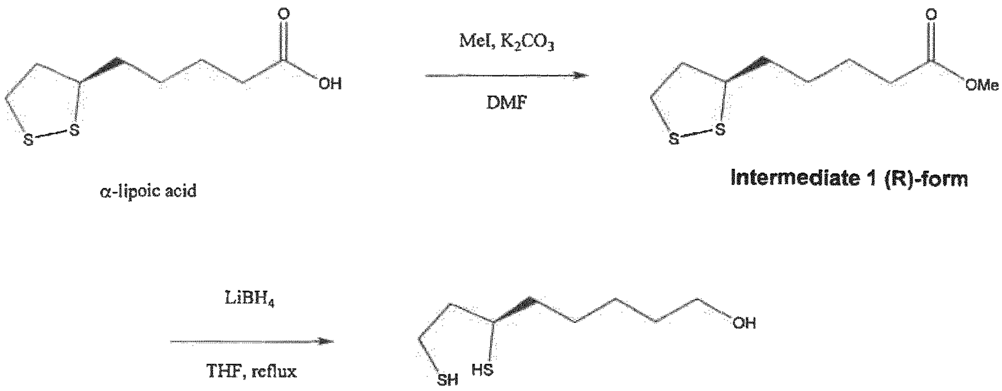


α-lipoic acid

**Intermediate 1 (R)-form**

**Compound 1 (R)-form**

Methyl (*R*)-5-(1,2-dithiolan-3-yl)pentanoate (Intermediate 1)

To a solution of α-lipoic acid (2.00 g, 9.69 mmol) in *N*,*N*-dimethylformamide (20 ml) were added methyl iodide (0.728 ml, 11.7 mmol) and potassium carbonate (2.68 g, 19.4 mmol) at room temperature under an argon atmosphere, and the mixture was stirred at room temperature overnight. The reaction mixture was filtered to remove potassium carbonate. The potassium carbonate was washed with ethyl acetate. The combined organic layer was washed with saturated brine, and dried over anhydrous magnesium sulfate. The mixture was filtered, and the filtrate was concentrated under reduced pressure, and the resulting residue was purified by silica gel column chromatography (hexane/ethyl acetate=10/1) to give the title Intermediate 1 (1.69 g, 79%) as a yellow oil.

| | |
|---|---|
| **Methyl (*R*)-5-(1,2-dithiolan-3-yl)pentanoate (Intermediate 1)** 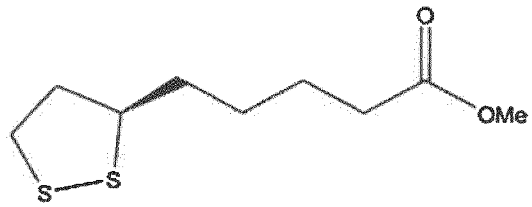 | δ $^1H$-NMR (400 MHz , $CDCl_3$)<br>δ 1.38-1.56 (m, 2H) , 1.62-1.78 (m, 4H) , 1.87-1.96 (m, 1H) , 2.33 (t, J = 7.8Hz, 2H), 2.42 - 2.51 (m, 1 H), 3.08-3.22 (m, 2 H), 3.53-3.61 (m, 1 H), 3.67 (s, 3H) |

(*R*)-6,8-Dimercaptooctan-1-ol (Compound 1)

To a solution of methyl (*R*)-5-(1,2-dithiolan-3-yl)pentanoate Intermediate 1 (796 mg, 3.63 mmol) in anhydrous tetrahydrofuran (10 ml) was added lithium borohydride (94.8 mg, 4.35 mmol) at room temperature under an argon atmosphere. The reaction mixture was refluxed for 1.5 hours, and then to the reaction mixture was further added lithium borohydride (23.7 mg, 1.09 mmol). The reaction mixture was refluxed for 1 hour, and then cooled to room temperature. Thereto were added 1 M hydrochloric acid and water to give a mixture of pH5 to 6. The mixture was extracted with ethyl acetate. The ethyl acetate layer was washed with saturated brine, and dried over anhydrous magnesium sulfate. The mixture was filtered, and the filtrate was concentrated under reduced pressure, and the resulting residue was purified by silica gel column chromatography (hexane/ethyl acetate=2/1) to give the title Compound 1 (625 mg, 89%) as a colorless oil.

| | |
|---|---|
| **(*R*)-6,8-Dimercaptooctan-1-ol (Compound 1)** 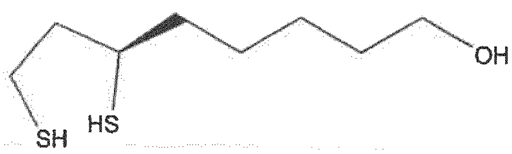 | δ $^1H$-NMR (400 MHz , $CDCl_3$)<br>δ 1.25-1.81 (m, 9H) , 1. 87-1. 97 (m, 1H) , 2. 62-2. 8 0 (m, 2H) , 2. 88-2. 98 (m, 1H) , 3.66 (t, J = 6. 9H z, 2H) |

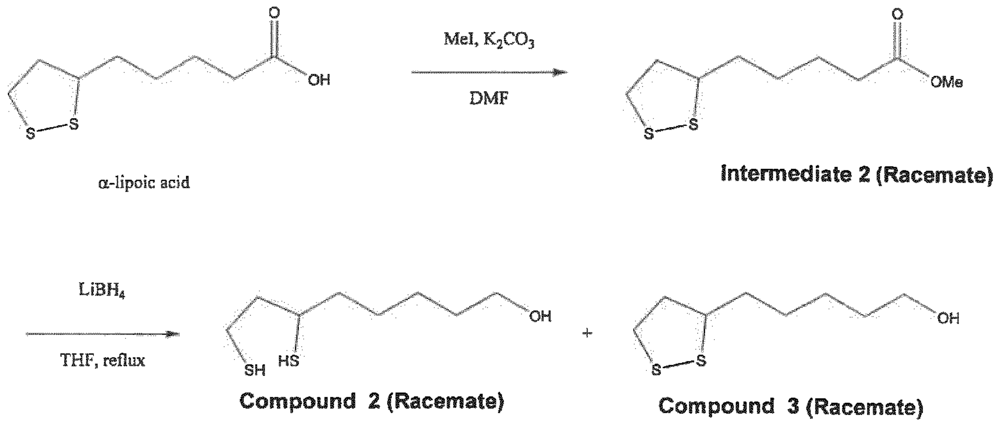


α-lipoic acid

Intermediate 2 (Racemate)

Compound 2 (Racemate) + Compound 3 (Racemate)

Methyl (1,2-dithiolan-3-yl)pentanoate (Intermediate 2)

To a solution of DL-α-lipoic acid (1.38 g, 6.69 mmol) in *N,N*-dimethylformamide (10 ml) were added methyl iodide (0.54 ml, 8.67 mmol) and potassium carbonate (1.39 g, 10.1 mmol) at room temperature, and the mixture was stirred at room temperature for 3 hours. Thereto was added water (40 ml), and the mixture was extracted with ethyl acetate (40 ml) twice. The ethyl acetate layer was washed with water (80 ml) and saturated brine (80 ml), and dried over anhydrous magnesium sulfate. The mixture was filtered, and the filtrate was concentrated under reduced pressure, and the resulting residue was purified by silica gel column chromatography (hexane/ethyl acetate=19/1) to give the title Intermediate 2 (3.07 g, 57%) as a yellow oil.

| **Methyl 5-(1,2-dithiolan-3-yl)pentanoate (Intermediate 2)** | δ $^1$H-NMR (400 MHz , $CDCl_3$) |
|---|---|
| 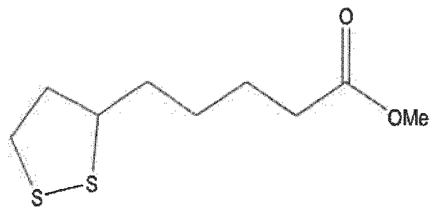 | δ 1.38-1. 54 (m, 2H) , 1. 59-1. 77(m, 4H), 1. 87-1. 95 (m, 1H), 2 , 33 (t, J = 7. 4Hz, 2H) , 2. 43-2. 51 (m, 1H) , 3. 09-3. 22 (m, 2H) , 3. 54-3. 67 (m, 1H) , 3. 67 (s, 3H) |

6,8-Dimercaptooctan-1-ol (Compound 2) and 5-(1,2-Dithiolan-3-yl)pentan-1-ol (Compound 3)

To a solution of methyl 5-(1,2-dithiolan-3-yl)pentanoate Intermediate 2 (0.52 g, 2.36 mmol) in tetrahydrofuran (5 ml) was added lithium borohydride (151 mg, 6.93 mmol) at room temperature, and the mixture was refluxed for 5 hours. Thereto were added water and 1 M hydrochloric acid to give a mixture of pH5 to 6. The mixture was extracted with ethyl acetate. The ethyl acetate layer was washed with saturated brine, and dried over anhydrous magnesium sulfate. The mixture was filtered, and the filtrate was concentrated under reduced pressure, and the resulting residue was purified by silica gel column chromatography (hexane/ethyl acetate=19/1 to 10/1) to give the title Compound 2 (150 mg, 33%) as a colorless oil, and the title Compound 3 (27.1 mg, 6%) as a yellow oil.

Compound 3 can be also obtained from Compound 2 by the following method (alternative method 1).

Compound 2 (300 mg, 1.55 mmol) was dissolved in tetrahydrofuran (200 ml), and thereto was added 1 N aqueous solution of sodium hydroxide (about 1 ml) to give a mixture of pH7 to 8, and then the mixture was stirred for at room temperature for 4 days. The reaction was concentrated under reduced pressure, and the residue was partitioned with water (20 ml) and chloroform (20 ml). The chloroform layer was washed with water (20 ml), 0.1 N hydrochloric acid (20 ml), water (20 ml), and saturated brine. The chloroform layer was dried over anhydrous magnesium sulfate, and the mixture was filtered, and the filtrate was concentrated under reduced pressure, and the resulting residue was purified by silica gel column chromatography (hexane/ethyl acetate=10/1) to give the title Compound 3 (183 mg, 62%) as a colorless oil.

| **6,8-Dimercaptooctan-1-ol (Compound 2)** | δ $^1$H-NMR (400 MHz , $CDCl_3$) |
|---|---|

(continued)

| | |
|---|---|
| 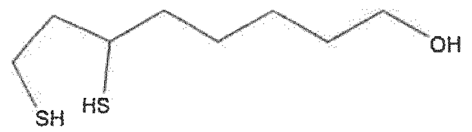 | δ 1. 30-1. 81 (m, 9H) , 1. 87-1. 97 (m, 1H) , 2. 62-2. 80 (m, 2H) , 2. 88-2. 98 (m, 1H) , 3. 66 (t, J = 6. 9H z, 2H) |
| **5-(1,2-Dithiolan-3-yl)pentan-1-ol (Compound 3)** 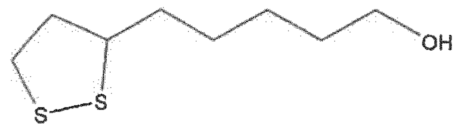 | δ $^1$H-NMR (400 MHz , $CDCl_3$) δ 1. 31-1. 81 (m, 8H) , 1 . 87-1. 97 (m, 1H) , 2. 43 - 2. 50 (m, 1H) , 3. 10-3. 22 (m, 2H) , 3. 57-3. 61 (m, 1H) , 3.66 (t, J = 6. 9H z, 2H) |

Compound 4 (114 mg, 35%) was obtained as a colorless oil from Compound 1 (320 mg, 1.55 mmol) according to the above synthetic method of Compound 3 (Alternative method 1).

| | |
|---|---|
| (*R*)-5-(1,2-Dithiolan-3-yl)pentan-1-ol (Compound 4) 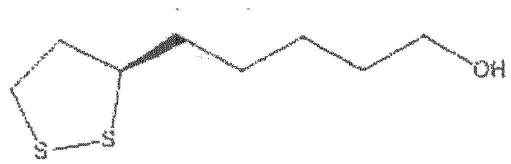 | δ $^1$H-NMR (400 MHz , $CDCl_3$) δ 1.32-1.80 (m, 8H) , 1.87-1.97 (m, 1H) , 2.43-2.5 0 (m, 1H) , 3.09-3.22 (m, 2H) , 3.54-3.61 (m, 1H) , 3.67 (q, J = 6.9 Hz, 3H) |

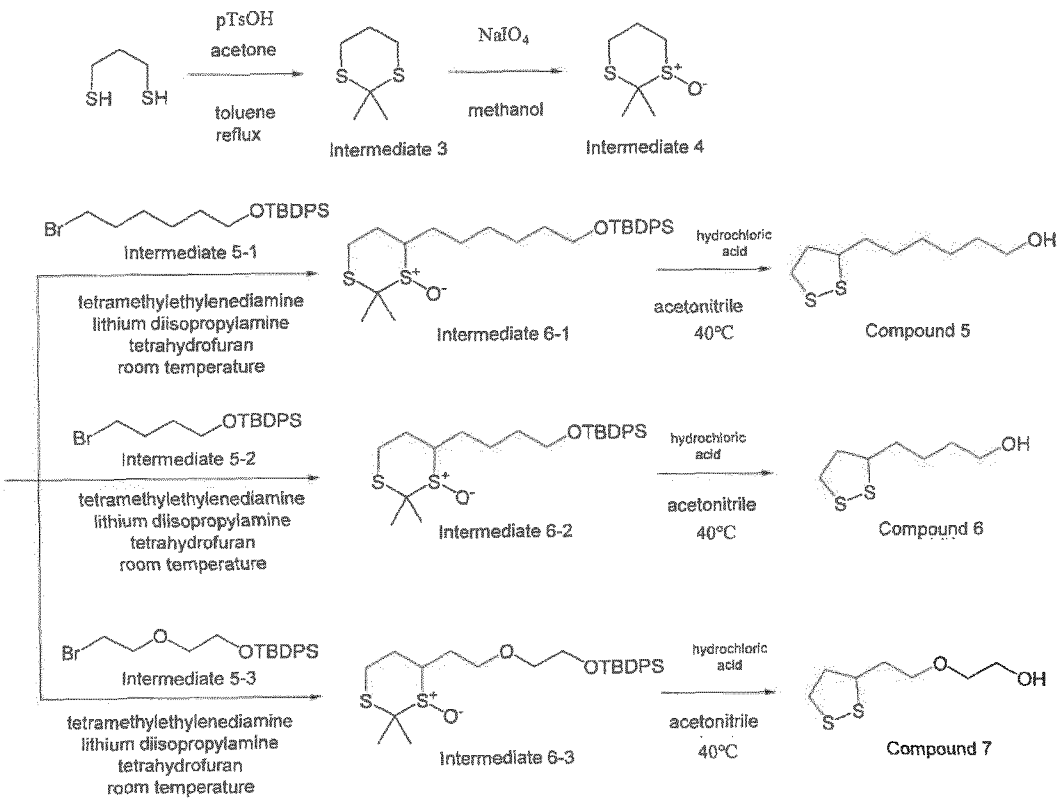


2,2-Dimethyl-1,3-dithiane (Intermediate 3)

To toluene (400mL) were added 1,3-propanedithiol(20 g, 212.3 mmol), acetone (13.57 g, 233.6 mmol) and a catalytic amount of p-toluenesulfonic acid, and the mixture was refluxed using Dean-Stark for 12 hours. The reaction mixture was allowed to cool to room temperature, and the resulting mixture was washed sequentially with 5% aqueous solution of sodium hydroxide (100 ml, twice), water (100 ml), and saturated brine (100 ml). The organic layer was dried

over anhydrous magnesium sulfate, and the solvent was removed under reduced pressure to give the title Intermediate 3 (20.7 g, 66%) as a colorless oil.

| | |
|---|---|
| 2,2-Dimethyl-1,3-dithiane (Intermediate 3)<br>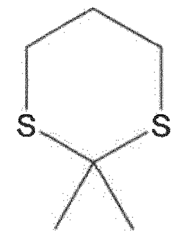 | 1H-NMR(300MHz, $CDCl_3$)<br>δ 1.72 (s, 6H), 1.92-2.06 (m, 2H), 2.87-2.96 (m, 4H). |

2,2-Dimethyl-1,3-dithian-1-ium-1-olate (Intermediate 4)

A solution of 2,2-dimethylmethyl-1,3-dithiane Intermediate 3 (20.7 g, 139.9 mmol) in methanol (1500 ml) was cooled to -5 °C under a nitrogen atmosphere, and thereto was added dropwise an aqueous solution of sodium periodate (29.9 g, 139.9 mmol) (250 ml) at a temperature maintained at -5 °C. The reaction mixture was stirred for 30 minutes at 20 °C or less, and then filtered and washed with methylene chloride (200 ml, 3 times). The filtrate was concentrated under reduced pressure, and the resulting residue was purified by reversed-phase chromatography (Column, C18 silica gel; acetonitrile/ water, 10% to 50%). Fractions containing the target compound were collected, and the resulting mixture was concentrated under reduced pressure, and the resulting residue was extracted with methylene chloride (1000 ml, 3 times). The combined organic layer was washed with saturated brine (4000 ml), dried over anhydrous sodium sulfate, and the solvent was removed under reduced pressure to give the title Intermediate 4 (20.8 g, 78%) as a pale yellow oil.

| | |
|---|---|
| 2,2-Dimethyl-1,3-dithian-1-ium-1-olate (Intermediate 4)<br>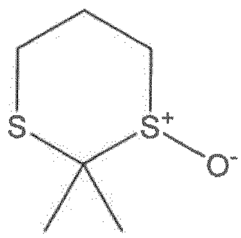 | $^1$H-NMR (300MHz, $CDCl_3$)<br>δ1.59 (s, 3H), 1.67 (s, 3H), 2.15-2.32 (m, 1H), 2.37-2.38 (m, 2H), 2.64-2.80 (m, 2H), 3.03-3.10 (m, 1H).<br>MS = 165 $(M + H)^+$ |

[(6-Bromohexyl)oxy](*tert*-butyl)diphenylsilane (Intermediate 5-1)

To a solution of 6-bromo-1-hexanol (4.2 g, 23.2 mmol) in methylene chloride (50 ml) were sequentially added 1H-imidazole (2.37 g, 34.8 mmol), *tert*-butyldiphenylsilyl chloride (9.56 g, 34.8 mmol) under a nitrogen atmosphere. The reaction was stirred at room temperature overnight. To the reaction was added water (150 ml), and the mixture was extracted with methylene chloride (150 ml, 3 times). The organic layer was washed with saturated brine (450 ml, 3 times), dried over anhydrous sodium sulfate, and the solvent was removed under reduced pressure. The resulting residue was purified by silica gel column chromatography (ethyl acetate/petroleum ether, 0 to 8%) to give the title Intermediate 5-1 (8.9 g, 91%) as an oil.

| | |
|---|---|
| [(6-Bromohexyl)oxy] (*tert*-butyl)diphenylsilane (Intermediate 5-1)<br>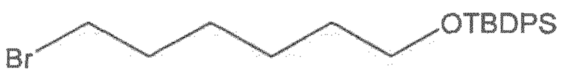 | $^1$H-NMR(300MHz, $CDCl_3$)<br>δ1.09 (s, 9H), 1.37-1.49 (m, 4H), 1.56-1.68 (m, 2H), 1.88-1.87 (m, 2H), 3.42 (t, J = 6.0Hz, 2H), 3.70 (t, J = 6.4Hz, 2H), 7.35-7.50 (m, 6H), 7.62-7.79 (m, 4H). |

[(4-Bromobutyl)oxy](*tert*-butyl)diphenylsilane (Intermediate 5-2)

The title Intermediate 5-2 (3.6 g, 51%) was obtained as a colorless oil from 4-bromo-1-butanol (2.75 g, 18.0

mmol) according to the above synthetic method of Intermediate 5-1.

| | |
|---|---|
| [(4-Bromobutyl)oxy](*tert*-butyl)diphenylsilane (Intermediate 5-2) 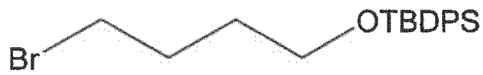 | $^1$H-NMR(400MHz, $CDCl_3$) δ1.09 (s, 9H), 1.70-1.77 (m, 2H), 2.01-2.08 (m, 2H), 3.44-3.47 (m, 2H), 3.71-3.74 (m, 2H), 7.40-7.49 (m, 6H), 7.67-7.77 (m, 4H). |

[2-(2-Bromoethoxy)ethoxy] (*tert*-butyl)diphenylsilane (Intermediate 5-3)

The title Intermediate 5-3 (10 g, 92%) was obtained as a yellow oil from 2-(2-bromoethoxy) ethanol (4.5 g, 26.6 mmol) according to the above synthetic method of Intermediate 5-1.

| | |
|---|---|
| [2-(2-Bromoethoxy)ethoxy](*tert*-butyl) diphenylsilane (Intermediate 5-3) 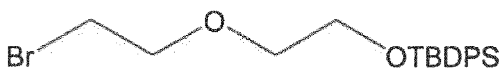 | $^1$H-NMR(400MHz, $CDCl_3$) δ1.08 (s, 9H), 3.46 (t, J = 6.3Hz, 2H), 3.65 (dd, J = 5.7, 4.4Hz, 2H),3.84 (ddd, J = 6.3, 5.5, 3.1Hz, 4H), 7.37-7.50 (m, 6H), 7.72 (dt, J = 6.5, 1.6Hz, 4H). |

6-2-{2-[(*tert*-Butyldiphenylsilyl)oxy]hexyl}-2,2-dimethyl-1,3-dithian-1-ium-1-olate (Intermediate 6-1)

To a solution of 2,2-dimethyl-1,3-dithian-1-ium-1-olate Intermediate 4 (1.74 g, 10.6 mmol) in tetrahydrofuran (12 ml) were sequentially added tetramethylethylenediamine (1.85 g, 15.9 mmol) and 2M solution of lithium diisopropylamide-tetrahydrofuran (7.94 ml, 15.9 mmol) under a nitrogen atmosphere at -78 °C. The mixture was stirred at -78 °C for 20 minutes. To the reaction mixture was added [(6-bromohexyl)oxy](*tert*-butyl)diphenylsilane Intermediate 5-1 (4.89 g, 11.7 mmol) at -78 °C, and the mixture was further stirred for 20 minutes. The reaction was brought to room temperature and stirred for 90 minutes. To the reaction mixtuire was added an aqueous solution of ammonium chloride (150 ml) at room temperature, and the mixture was extracted with ethyl acetate (150 ml, 3 times). The organic layer was washed with saturated brine (450 ml, 3 times), dried over anhydrous sodium sulfate, and the solvent was removed under reduced pressure. The resulting residue was purified by silica gel column chromatography (ethyl acetate/petroleum ether, 0 to 48%) to give the title Intermediate 6-1 (3.2 g, 60%) as a colorless oil.

| | |
|---|---|
| 6-2-{2-[(*tert*-Butyldiphenylsilyl)oxy]hexyl}-2,2-dimethyl-1,3-dithian-1-ium-1-olate (Intermediate 6-1) 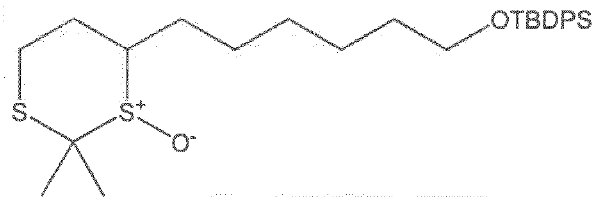 | 1H-NMR(400MHz, $CDCl_3$) δ1.07 (s, 9H), 1.28-1.44 (m, 5H), 1.52-1.67 (m, 12H), 1.93-2.22 (m, 1H), 2.33-2.66 (m, 2H), 2.79-2.92 (m, 1H), 3.64-3.70 (m, 2H), 7.38-7.46 (m, 6H), 7.65-7.72 (m, 4H). MS = 525 $(M+Na)^+$ |

6-{4-[(*tert*-Butyldiphenylsilyl)oxy]butyl}-2,2-dimethyl-1,3-dithian-1-ium-1-olate (Intermediate 6-2)

The title Intermediate 6-2 (3.43 g, 75%) was obtained as a colorless oil from 2,2-dimethyl-1,3-dithian-1-ium-1-olate Intermediate 4 (1.58 g, 9.61 mmol) and [(4-bromobutyl)oxy](*tert*-butyl)diphenylsilane Intermediate 5-2 according to the above synthetic method of Intermediate 6-1.

| | |
|---|---|
| 6-{4-[(*tert*-Butyldiphenylsilyl)oxy]butyl}-2,2-dimethyl-1,3-dithian-1-ium-1-olate (Intermediate 6-2)<br>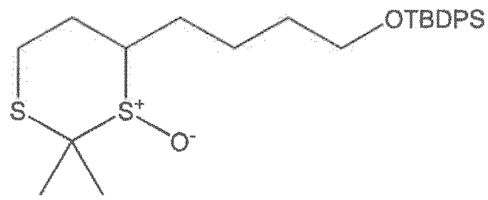 | 1H-NMR(400MHz, $CDCl_3$) δ1.07 (s, 9H), 1.23-1.32 (m, 1H), 1.39 (s, 1), 1.61(brs, 5H), 1.67 (brs, 2H), 1.71 (s, 1H), 1.93-2.04 (m, 1H), 2.10 (s, 1H), 2.33-2.43 (m, 2H), 2.52-2.63 (m, 1H), 2.76-2.90 (m, 1H), 3.66-3.79 (m, 2H), 7.36-7.49 (m, 6H), 7.64-7.72 (m, 4H).<br>MS = 497.1 $(M+Na)^+$ |

6-(2-{2-[(*tert*-Butyldiphenylsilyl)oxy]ethoxy}ethyl)-2,2-dimethyl-1,3-dithian-1-ium-1-olate (Intermediate 6-3)

The title Intermediate 6-3 (5.5 g, 83%) was obtained as a colorless oil from 2,2-dimethyl-1,3-dithian-1-ium-1-olate Intermediate 4 (2.2 g, 13.4 mmol) and [2-(2-bromoethoxy)ethoxy] (*tert*-butyl)diphenylsilane Intermediate 5-3 (6.0 g, 14.7 mmol) according to the above synthetic method of Intermediate 6-1 described above.

| | |
|---|---|
| 6-(2-{2-[(*tert*-Butyldiphenylsilyl)oxy]ethoxy}ethyl)-2,2-dimethyl-1,3-dithian-1-ium-1-olate (Intermediate 6-3)<br>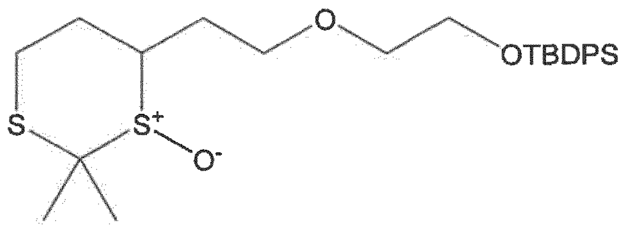 | 1H-NMR(400MHz, $CDCl_3$)<br>δ1.05 (s, 9H), 1.55 (s, 3H), 1.63 (s, 3H), 1.73-1.81 (m, 1H), 1.94-2.02 (m, 1H), 2.23-2.29 (m, 1H), 2, 37-2.54 (m, 2H), 2.70-2.80 (m, 2H), 3.47-3.74 (m, 4H), 3.81-3.78 (m, 2H), 7.33-7.47 (m, 6H), 7.65-7.72 (m, 4H).<br>MS = 491.1 $(M+ Na)^+$ |

6-(1,2-Dithiolan-3-yl)hexan-1-ol (Compound 5)

To a solution of 6-(2-{2-[(*tert*-butyldiphenylsilyl)oxy]ethoxy}ethyl)-2,2-dimethyl-1,3-dithian-1-ium-1-olate Intermediate 6-1 (3.3 g, 66.6 mmol) in acetonitrile (33 ml) was added dropwise 2 M hydrochloric acid (1.64 ml, 3.28 mmol) under a nitrogen atmosphere, and the mixture was stirred overnight at 40 °C. To the reaction mixture was added an aqueous solution of sodium hydrogen carbonate (10 ml). Then the mixture was extracted with methylene chloride (100 ml, 3 times). The organic layer was washed with saturated brine (400 ml), and dried over anhydrous sodium sulfate. The solvent was removed under reduced pressure. The resulting residue was purified by reversed-phase chromatography (Column, C18 silica gel; acetonitrile/ water, 5% to 95%, 45 minutes). Fractions containing the target compound were collected, and the resulting mixture was concentrated under reduced pressure, and the resulting residue was extracted with methylene chloride (100 ml, 3 times). The organic layer was washed with saturated brine (400 ml) and dried over anhydrous sodium sulfate. The solvent was removed under reduced pressure to give the title Compound 5 (320 mg, 24%) as a pale yellow oil.

| | |
|---|---|
| 6-(1,2-Dithiolan-3-yl)hexan-1-ol (Compound 5)<br>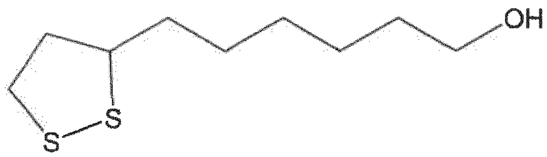 | 1H-NMR(400MHz, $CDCl_3$) δ1.32-1.76 (m, 11H), 1. 89-1. 97 (m, 1H), 2.45-2.52 (m, 1H), 3.11-3.24 (m, 2H), 3.56-3.68 (m, 3H).<br>MS = 207 $(M+H)^+$ |

4-(1,2-Dithiolan-3-yl)butan-1-ol (Compound 6)

The title Compound 6 (413 mg, 36%) was obtained as a yellow oil from 6-{4-[(*tert*-butyldiphenylsilyl)oxy]butyl}-2,2-dimethyl-1,3-dithian-1-ium-1-olate Intermediate 6-2 (2.93 g, 6.2 mmol) according to the above synthetic method of Compound 5.

| | |
|---|---|
| 4-(1,2-Dithiolan-3-yl)butan-1-ol (Compound 6) 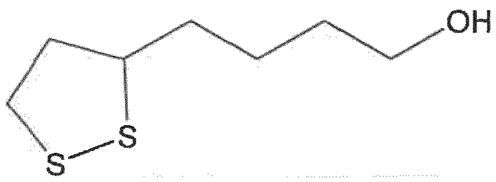 | 1H-NMR(400MHz, $CDCl_3$) δ1.46-1.79 (m, 7H), 1.90-1.98 (m, 1H), 2.45-2.55 (m, 1H) 3.10-3.26 (m, 2H), 3.57-3.71 (m, 3H). MS = 178 ($M^+$) |

2-[2-(1,2-Dithiolan-3-yl)ethoxy]ethan-1-ol (Compound 7)

The title Compound 7 (718 mg, 36%) was obtained as as a yellow oil from 6-(2-{2-[(*tert*-butyldiphenylsilyl)oxy]ethoxy}ethyl)-2,2-dimethyl-1,3-dithian-1-ium-1-olate Intermediate 6-3 (5.0 g, 10.2 mmol) according to the above synthetic method of Compound 5.

| | |
|---|---|
| 2-[2-(1,2-Dithiolan-3-yl)ethoxy]ethan-1-ol (Compound 7) 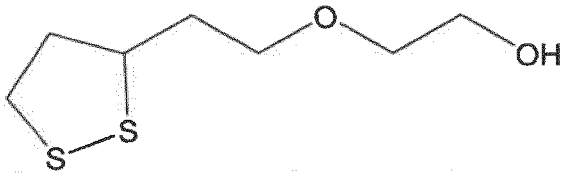 | 1H-NMR(400MHz, $CDCl_3$) δ1.90-2.08 (m, 3H), 2.49-2.57 (m, 1H), 3.12-3.24 (m, 2H) 3.56-3.71 (m, 4H), 3.71-3.80 (m, 3H). MS = 212 $(M+NH_4)^+$ |

[Reference Pharmacological test 1]

The effect of lipoic acid choline ester (EV06) on the lens elasticity was examined. The tests were conducted with reference to the methods described in InvestOphthalmol Vis Sci, 57, 2851-2863, 2016. EV06 is a compound represented by the following formula:

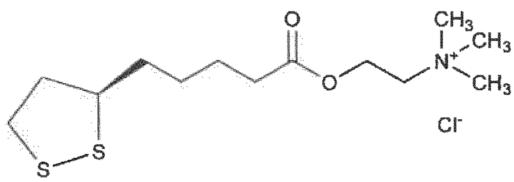

(Preparation of Test sample)

1) Preparation of Vehicle

A vehicle comprising 0.1% (w/v) of ethyl pyruvate, 0.269% (w/v) of sodium dihydrogenphosphate monohydrate ($NaH_2PO_4.H_2O$), 0.433% (w/v) of disodium hydrogenphosphate ($Na_2HPO_4$), 0.2% (w/v) of hydroxypropyl methylcellulose, 0.5% (w/v) of NaCl, and purified water (appropriate amount) was prepared.

2) Preparation of EV06 sample

EV06 was sonicated with the addition of the vehicle to prepare a 5% (w/v) suspension. The resulting 5% (w/v) suspension was diluted with the vehicle to prepare a 1.5% (w/v) solution. Further, the resulting 1.5% (w/v) solution was diluted with the vehicle to prepare a 0.5% (w/v) solution. The total amount of each sample to be used in one day was prepared before use.

(Test method)

1) Each test sample (2.5 μL/eye) was instilled into the right eye of 8-month-old C57BL/6J mice with a Pipetman 3

times per day (around 9: 00, 13: 00 and 17: 00) for 15 to 17 days.
2) After the final instillation, the mice were euthanized by carbon dioxide inhalation, and then the eyeballs were extracted and rinsed with Hank's balanced salt solution (HBSS) .
3) The sclera near the optic nerve was cut with a razor, the lens was removed through the incision, and the removed lens was immersed in HBSS.
4) The lens was placed on a glass slide, and an all-in-one fluorescence microscope BZ-9000 (Keyence) was used to capture an image of the lens (Image a).
5) Next, one cover glass (Corning(registered trade mark) 22 × 22 mm Square) was placed on the lens, and an image in which the thickness of the lens changed due to the weight was similarly captured (Image b).
6) A change in the lens diameter was calculated from Equation 1 wherein the lens diameter of Image a is subtracted from the lens diameter of Image b, as described below. Then, the lens elasticity improvement of each sample group compared with the vehicle control group was calculated from Equation 2 described below. The mean of the vehicle control group was based on 6 eyes and the mean of each EV06 sample group was based on 12 eyes.

(Equation 1)
Change in lens diameter =
Lens diameter in Image b of each test sample - Lens diameter in Image a of each test sample

(Equation 2)
Lens elasticity improvement of each sample group =
Mean change in lens diameter of each EV06 sample group - Mean change in lens diameter of Vehicle control group

(Results)

The results are shown in Table 1.

[Table 1]

| | Lens elasticity improvement (μm) |
|---|---|
| 0.5% EV06 sample | 28.8 |
| 1.5% EV06 sample | 47.3 |
| 5% EV06 sample | 48.7 |

As shown in Table 1, all of the 0.5%, 1.5%, and 5% EV06 groups showed the increased lens diameter compared with the vehicle control group, which confirms that EV06 has an elasticity improving effect.

[Pharmacological test-1]

The effect of 3 times-daily instillation of Compound 1 for 2 weeks on the lens elasticity was examined. The tests were conducted with reference to the methods described in InvestOphthalmol Vis Sci, 57, 2851-2863, 2016.

(Preparation of Test sample)

1) Preparation of Vehicle

A vehicle comprising 1.0% (w/v) of polyoxyl 35 castor oil (hereinafter also referred to as "CO35"), 0.1% (w/v) of ethyl pyruvate, 0.269% (w/v) of sodium dihydrogenphosphate monohydrate ($NaH_2PO_4 . H_2O$), 0.433% (w/v) of disodium hydrogenphosphate ($Na_2HPO_4$), 0.2% (w/v) of hydroxypropyl methylcellulose, 0.5% (w/v) of NaCl, and purified water (appropriate amount) was prepared.

2) Preparation of Compound 1 sample

Compound 1 was stirred with the addition of the vehicle to give a 0.03% (w/v) solution. After preparation, it was refrigerated.

3) Preparation of EV06 sample

EV06 was stirred with the addition of the vehicle to give a 0.03% (w/v) solution, which was for comparison in the test for Compound 1. After preparation, it was refrigerated.

.(Test method)

1) Each test sample (2.5 μL/eye) was instilled into both eyes of 9-month-old C57BL/6J mice with a Pipetman 3 times per day (around 9: 00, 13: 00 and 17: 00) for 14 days.
2) After the final instillation, the mice were euthanized by carbon dioxide inhalation, and then the eyeballs were extracted and rinsed with Hank's balanced salt solution (HBSS).
3) The sclera near the optic nerve was cut with a razor, the lens was removed through the incision, and the removed lens was immersed in HBSS.
4) The lens was placed on a glass slide, and an all-in-one fluorescence microscope BZ-9000 (Keyence) was used to capture an image of the lens (Image a).
5) Next, one cover glass (Corning(registered trade mark) 22 × 22 mm Square) was placed on the lens, and an image in which the thickness of the lens changed due to the weight was similarly captured (Image b).
6) A change in the lens diameter was calculated from the following Equation 1 wherein the lens diameter of Image a is subtracted from the lens diameter of Image b. Then, the lens elasticity improvement of each sample group compared with the vehicle control group was calculated from Equation 2 described below. The mean value of the vehicle control group was based on 8 eyes, and the mean value of each of the sample groups of Compound 1 and EV06 was based on 10 eyes.

(Equation 1)
Change in lens diameter =
Lens diameter in Image b of each test sample - Lens diameter in Image a of each test sample

(Equation 2)
Lens elasticity improvement of each sample group =
Mean change in lens diameter of each Test sample group - Mean change in lens diameter of Vehicle control group

(Results)

The results are shown in Table 2.

[Table 2]

| | Lens elasticity improvement (μm) |
|---|---|
| 0.03% Compound 1 sample | 53.8 |
| 0.03% EV06 sample | 3.6 |

As shown in Table 2, the 0.03% Compound 1 sample group showed a large increase in lens diameter compared to the vehicle control group, indicating a potent elasticity improving effect. On the other hand, the 0.03% EV06 sample group showed little increase in lens diameter compared to the vehicle control group, indicating no improvement in lens elasticity.

[Pharmacological test-2]

The effect of once-daily instillation of Compound 1 for 2 weeks on the lens elasticity was examined.

1) Preparation of Vehicle

A vehicle comprising 1.0% (w/v) of CO35, 0.1% (w/v) of ethyl pyruvate, 0.269% (w/v) of sodium dihydrogenphosphate monohydrate ($NaH_2PO_4 . H_2O$), 0.433% (w/v) of disodium hydrogenphosphate ($Na_2HPO_4$), 0.2% (w/v) of hydroxypropyl methylcellulose, 0.5% (w/v) of NaCl, and purified water (appropriate amount) was prepared.

2) Preparation of Compound 1 sample

To Compound 1 was added the vehicle to give a 0.1% (w/v) solution. The resulting 0.1% (w/v) solution was diluted with the vehicle to give a 0.03% (w/v) solution. Further, the resulting 0.03% (w/v) solution was diluted with the vehicle to give a 0.01% (w/v) solution. After preparation, all were frozen.

(Test method)

1) Each test sample (2.5 μL/eye) was instilled into both eyes of 9-month-old C57BL/6J mice with a Pipetman once per day (around 9: 00) for 14 days.
2) After the final instillation, the mice were euthanized by carbon dioxide inhalation, and then the eyeballs were extracted and rinsed with Hank's balanced salt solution (HBSS).
3) The sclera near the optic nerve was cut with a razor, the lens was removed through the incision, and the removed lens was immersed in HBSS.
4) The lens was placed on a glass slide, and an all-in-one fluorescence microscope BZ-9000 (Keyence) was used to capture an image of the lens (Image a).
5) Next, one cover glass (Corning(registered trade mark) 22 × 22 mm Square) was placed on the lens, and an image in which the thickness of the lens changed due to the weight was similarly captured (Image b).
6) A change in the lens diameter was calculated from Equation 1 wherein the lens diameter of Image a is subtracted from the lens diameter of Image b, as described below. Then, the lens elasticity improvement of each sample group compared with the vehicle control group was calculated from Equation 2 described below. The mean value of the vehicle control group was based on 9 eyes, and the mean value of each of the sample groups of Compound 1 was based on 10 eyes.

(Equation 1)
Change in lens diameter =
Lens diameter in Image b of each test sample - Lens diameter in Image a of each test sample

(Equation 2)
Lens elasticity improvement of each sample group =
Mean change in lens diameter of each Test sample group - Mean change in lens diameter of Vehicle control group

(Results)

The results are shown in Table 3.

[Table 3]

| | Lens elasticity improvement (μm) |
|---|---|
| 0.01% Compound 1 sample | 17.5 |
| 0.03% Compound 1 sample | 53.0 |

(continued)

| | Lens elasticity improvement (μm) |
|---|---|
| 0.1% Compound 1 sample | 63.3 |

As shown in Table 3, Compound 1 sample groups showed an elasticity improving effect at all of the concentrations of 0.01%, 0.03%, and 0.1%.

[Pharmacological test-3]

The effect of once-daily instillation of each of Compounds 1, 2 and 3 for 2 weeks on the lens elasticity was examined.

1) Preparation of Vehicle

A vehicle comprising 1.0% (w/v) of CO35, 0.1% (w/v) of ethyl pyruvate, 0.269% (w/v) of sodium dihydrogenphosphate monohydrate ($NaH_2$ $PO_4$ . $H_2O$), 0.433% (w/v) of disodium hydrogenphosphate ($Na_2HPO_4$), 0.2% (w/v) of hydroxypropyl methylcellulose, 0.5% (w/v) of NaCl, and purified water (appropriate amount) was prepared.

2) Preparation of Compounds 1, 2, and 3 samples

Each of Compounds 1 and 2 was stirred with the addition of the vehicle to give a 0.1% (w/v) solution.
To Compound 3 was added the vehicle to give a 0.1% (w/v) suspension. The resulting 0.1% (w/v) suspension was diluted with the vehicle to give a 0.03% (w/v) solution. Further, the resulting 0.03% (w/v) solution was diluted with the vehicle to give a 0.01% (w/v) solution.
After preparation, all were frozen.

(Test method)

1) Each test sample (2.5 μL/eye) was instilled into both eyes of 8-month-old C57BL/6J mice with a Pipetman once per day (around 9: 00) for 14 days.
2) After the final instillation, the mice were euthanized by carbon dioxide inhalation, and then the eyeballs were extracted and rinsed with Hank's balanced salt solution (HBSS) .
3) The sclera near the optic nerve was cut with a razor, the lens was removed through the incision, and the removed lens was immersed in HBSS.
4) The lens was placed on a glass slide, and an all-in-one fluorescence microscope BZ-9000 (Keyence) was used to capture an image of the lens (Image a).
5) Next, one cover glass (Corning(registered trade mark) 22 × 22 mm Square) was placed on the lens, and an image in which the thickness of the lens changed due to the weight was similarly captured (Image b).
6) A change in the lens diameter was calculated from Equation 1 wherein the lens diameter of Image a is subtracted from the lens diameter of Image b, as described below. Then, the lens elasticity improvement of each sample group compared with the vehicle control group was calculated from Equation 2 described below. The mean value of each group was based on 10 eyes.

(Equation 1)
Change in lens diameter =
Lens diameter in Image b of each test sample - Lens diameter in Image a of each test sample

(Equation 2)
Lens elasticity improvement of each sample group =
Mean change in lens diameter of each Test sample group - Mean change in lens diameter of Vehicle control group

(Results)

The results are shown in Table 4.

[Table 4]

| | Lens elasticity improvement (μm) |
|---|---|
| 0.03% Compound 1 sample | 55.5 |
| 0.03% Compound 2 sample | 42.2 |
| 0.01% Compound 3 sample | 21.5 |
| 0.03% Compound 3 sample | 47.3 |
| 0.1% Compound 3 sample | 60.1 |

As shown in Table 4, Compound 3 sample groups showed an elasticity improving effect at all of the concentrations of 0.01%, 0.03%, and 0.1%. The 0.03% Compound 2 sample group also showed a potent elasticity improving effect.

[Pharmacological test-4]

The effect of once-daily instillation of each of Compounds 3 and 4 for 2 weeks on the lens elasticity was examined.

1) Preparation of Vehicle

A vehicle comprising 1.0% (w/v) of CO35, 0.1% (w/v) of ethyl pyruvate, 0.269% (w/v) of sodium dihydrogenphosphate monohydrate ($NaH_2PO_4 . H_2O$), 0.433% (w/v) of disodium hydrogenphosphate ($Na_2HPO_4$), 0.2% (w/v) of hydroxypropyl methylcellulose, 0.5% (w/v) of NaCl, and purified water (appropriate amount) was prepared.

2) Preparation of Compounds 3 and 4 samples

Compound 3 was stirred with the addition of the vehicle to give a 0.03% (w/v) suspension.
Compound 4 was stirred with the addition of the vehicle to give a 0.1% (w/v) suspension. The resulting 0.1% (w/v) suspension was diluted with the vehicle to give a 0.03% (w/v) suspension. Further, the resulting 0.03% (w/v) suspension was diluted with the vehicle to give a 0.01% (w/v) suspension.
After preparation, all were refrigerated.

(Test method)

1) Each test sample (2.5 μL/eye) was instilled into both eyes of 8-month-old C57BL/6J mice with a Pipetman once per day (around 9: 00) for 14 days.
2) After the final instillation, the mice were euthanized by carbon dioxide inhalation, and then the eyeballs were extracted and rinsed with Hank's balanced salt solution (HBSS) .
3) The sclera near the optic nerve was cut with a razor, the lens was removed through the incision, and the removed lens was immersed in HBSS.
4) The lens was placed on a glass slide, and an all-in-one fluorescence microscope BZ-9000 (Keyence) was used to capture an image of the lens (Image a).
5) Next, one cover glass (Corning(registered trade mark) 22 × 22 mm Square) was placed on the lens, and an image in which the thickness of the lens changed due to the weight was similarly captured (Image b).
6) A change in the lens diameter was calculated from Equation 1 wherein the lens diameter of Image a is subtracted from the lens diameter of Image b, as described below. Then, the lens elasticity improvement of each sample group compared with the vehicle control group was calculated from Equation 2 described below. The mean value of each group was based on 10 eyes.

(Equation 1)

Change in lens diameter =
Lens diameter in Image b of each test sample - Lens diameter in Image a of each test sample

(Equation 2)

Lens elasticity improvement of each sample group =
Mean change in lens diameter of each Test sample group - Mean change in lens diameter of Vehicle control group

(Results)

The results are shown in Table 5.

[Table 5]

| | Lens elasticity improvement (μm) |
|---|---|
| 0.03% Compound 3 sample | 39.9 |
| 0.01% Compound 4 sample | 31.1 |
| 0.03% Compound 4 sample | 60.2 |
| 0.1% Compound 4 sample | 66.7 |

As shown in Table 5, Compound 4 sample groups showed an elasticity improving effect at all of the concentrations of 0.01%, 0.03%, and 0.1%.

[Pharmacological test-5]

The effect of once-daily instillation of each of Compounds 5, 6, and 7 for 2 weeks on the lens elasticity was examined.

1) Preparation of Vehicle

A vehicle comprising 1.0% (w/v) of CO35, 0.1% (w/v) of ethyl pyruvate, 0.269% (w/v) of sodium dihydrogenphosphate monohydrate ($NaH_2PO_4 . H_2O$), 0.433% (w/v) of disodium hydrogenphosphate ($Na_2HPO_4$), 0.2% (w/v) of hydroxypropyl methylcellulose, 0.5% (w/v) of NaCl, and purified water (appropriate amount) was prepared.

2) Preparation of Compounds 5, 6, and 7 samples

Compound 5 was stirred with the addition of the vehicle to give a 0.1% (w/v) suspension. The resulting 0.1% (w/v) suspension was diluted with the vehicle to give a 0.03% (w/v) suspension.

Compound 6 was stirred with the addition of the vehicle to give a 0.1% (w/v) suspension. The resulting 0.1% (w/v) a suspension was diluted with the vehicle to give a 0.03% (w/v) suspension.

Compound 7 was stirred with the addition of the vehicle to give a 0.1% (w/v) suspension.

After preparation, all were refrigerated.

3) Preparation of EV06 sample

EV06 was stirred with the addition of the vehicle to give a 0.03% (w/v) solution, which was for comparison in the test for Compounds 5, 6 and 7. After preparation, it was refrigerated.

(Test method)

1) Each test sample (2.5 μL/eye) was instilled into both eyes of 8-month-old C57BL/6J mice with a Pipetman once per day (around 13: 30) for 14 days.
2) After the final instillation, the mice were euthanized by carbon dioxide inhalation, and then the eyeballs were extracted and rinsed with Hank's balanced salt solution (HBSS).
3) The sclera near the optic nerve was cut with a razor, the lens was removed through the incision, and the removed lens was immersed in HBSS.
4) The lens was placed on a glass slide, and an all-in-one fluorescence microscope BZ-9000 (Keyence) was used to capture an image of the lens (Image a).
5) Next, one cover glass (Corning(registered trade mark) 22 × 22 mm Square) was placed on the lens, and an image in which the thickness of the lens changed due to the weight was similarly captured (Image b).
6) A change in the lens diameter was calculated from Equation 1 wherein the lens diameter of Image a is subtracted from the lens diameter of Image b, as described below. Then, the lens elasticity improvement of each sample group compared with the vehicle control group was calculated from Equation 2 described below. The mean value of each group was based on 8 eyes.

(Equation 1)
Change in lens diameter =
Lens diameter in Image b of each test sample - Lens diameter in Image a of each test sample

(Equation 2)
Lens elasticity improvement of each sample group =
Mean change in lens diameter of each Test sample group - Mean change in lens diameter of Vehicle control group

(Results)

The results are shown in Table 6.

[Table 6]

| | Lens elasticity improvement (μm) |
|---|---|
| 0.03% Compound 5 sample | 22.3 |
| 0.1% Compound 5 sample | 26.6 |
| 0.03% Compound 6 sample | 40.0 |
| 0.1% Compound 6 sample | 58.9 |
| 0.1% Compound 7 sample | 41.4 |
| 0.03% EV06 sample | 3.1 |

As shown in Table 6, Compounds 5 and 6 sample groups showed a potent elasticity improving effect at the concentrations of 0.03% and 0.1%.
The 0.03% EV06 sample group showed no elasticity improving effect.
The 0.1% Compound 7 sample group showed a potent elasticity improving effect.

[Ocular irritation test-1]

(Preparation of sample)

A vehicle eye drop and a 0.03% (w/v) Compound 1 eye drop were prepared in the same manner as Preparation of Test sample in Pharmacological test-1.

(Test method)

The vehicle eye drop, and the 0.03% (w/v) Compound 1 eye drop were each instilled into one eye of a Japanese White rabbit at a dose volume of 50 μL/eye with pipette 10 times at 30-minute intervals. One hour after the final instillation, ocular irritation of anterior segment of the eye was evaluated using a slit lamp according to the McDonald-Shadduck method.

Observation for corneal epithelial disorder was conducted 3 hours after the final instillation.

The ocular irritation of anterior segment of the eye was scored according to the following criteria:
+1: slight; +2: moderate; +3: severe

(Test result)

The results are shown in Table 7. No irritation was observed in anterior segment of the eye treated with the 0.03% (w/v) Compound 1 eye drop.

[Table 7]

| Eye drop | | vehicle | 0.03% Compound 1 |
|---|---|---|---|
| Number of animals | | 3 | 3 |
| Irritation Score of anterior segment of the eye | Conjunctival hyperemia | - | - |
| | Conjunctival edema | - | - |
| | Discharge | - | - |
| | Anterior chamber flare | - | - |
| | Light reflex | - | - |
| | Iris | - | - |
| | Corneal opacity | - | - |
| | Corneal angiogenesis | - | - |
| | Corneal epithelial disorder | - | - |
| -: No noteworthy findings<br>Findings and score of the instilled eye at 1 hour after the final instillation were described. Observation for corneal epithelial disorder was conducted 3 hours after the final instillation. | | | |

(Discussion)

It is shown that the eye drop of Compound 1 is highly safe.

[Ocular irritation test-2]

(Preparation of sample)

A vehicle eye drop and 0.01% (w/v) and 0.1% (w/v) Compound 4 eye drops were prepared in the same manner as Preparation of Test sample in Pharmacological test-4.

(Test method)

The vehicle eye drop, and the 0.01% (w/v) and 0.1% (w/v) Compound 4 eye drops were each instilled into one eye of a Japanese White rabbit at a dose volume of 50 pL/eye with pipette 10 times at 30-minute intervals. One hour after the final instillation, ocular irritation of anterior segment of the eye was evaluated using a slit lamp according to the McDonald-Shadduck method. Observation for corneal epithelial disorder was conducted 3 hours after the final instillation.

The ocular irritation of anterior segment of the eye was scored according to the following criteria:
+1: slight; +2: moderate; +3: severe

(Test result)

The results are shown in Table 8. No irritation was observed in anterior segment of the eyes treated with the 0.01% (w/v) and 0.1% (w/v) Compound 4 eye drops.

[Table 8]

| Eye drop | | Vehicle | 0.01% Compound 4 | 0.1% Compound 4 |
|---|---|---|---|---|
| Number of animals | | 3 | 3 | 3 |
| Irritation Score of anterior segment of the eye | Conjunctival hyperemia | - | - | - |
| | Conjunctival edema | - | - | - |
| | Discharge | - | - | - |
| | Anterior chamber flare | - | - | - |
| | Light reflex | - | - | - |
| | Iris | - | - | - |
| | Corneal opacity | - | - | - |
| | Corneal angiogenesis | - | - | - |
| | Corneal epithelial disorder | - | - | - |
| -: No noteworthy findings<br>Findings and score of the instilled eye at 1 hour after the final instillation were described. Observation for corneal epithelial disorder was conducted 3 hours after the final instillation. | | | | |

(Discussion)

It is shown that the eye drops of Compound 4 are highly safe.

Industrial applicability

The compound/agent/composition of the present invention are useful for treating or preventing eye diseases such as presbyopia etc.

## Claims

1. An agent/composition for treating or preventing presbyopia, comprising, as an active ingredient, a compound represented by Formula [I]

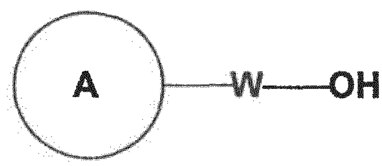 [I]

or a pharmaceutically acceptable salt thereof, wherein:

the A moiety is

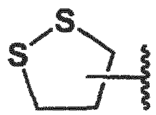 , 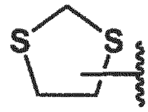 ,

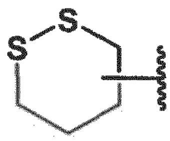 , 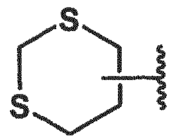 , 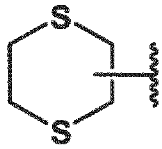

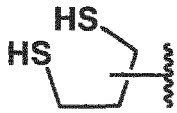 or 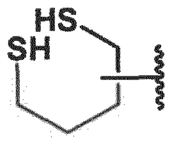 ,

wherein the A moiety may be optionally substituted with the same or different one to three $R^1$;
$R^1$, at each occurrence, is independently selected from the group consisting of -OH, halo, $C_{1-3}$ alkyl, and $C_{1-3}$ alkoxy;
W is $C_{1-10}$ alkylene; in which one or two methylene groups in the alkylene may each be independently replaced with a divalent group selected from the group consisting of O, $NR^2$ in which $R^2$ is H or $C_{1-3}$ alkyl, and $S(O)_{0-2}$; and in which the alkylene may be optionally substituted with at least one substituent independently selected from the group consisting of -OH, halo, $C_{1-3}$ alkyl, and $C_{1-3}$ alkoxy.

**2.** An agent/composition for treating or preventing an eye disease accompanied by a decrease in lens elasticity, comprising, as an active ingredient, a compound of Formula [I]

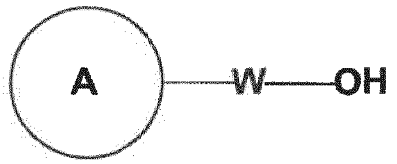 [I] ,

wherein the A moiety and W each has the same meaning as defined in Claim 1,
or a pharmaceutically acceptable salt thereof.

**3.** The agent/composition according to Claim 2, wherein the eye disease is accompanied by a decrease in accommodative function of the eye.

**4.** An agent/composition for treating or preventing an eye disease accompanied by a decrease in accommodative function of the eye, comprising, as an active ingredient, a compound of Formula [I]

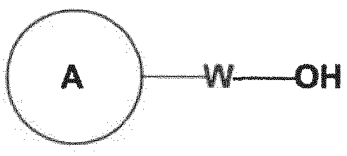 [I] ,

wherein the A moiety and W each has the same meaning as defined in Claim 1,
or a pharmaceutically acceptable salt thereof.

**5.** The agent/composition according to any one of 2 to 4, wherein the eye disease is presbyopia.

**6.** A composition comprising a compound of Formula [I]

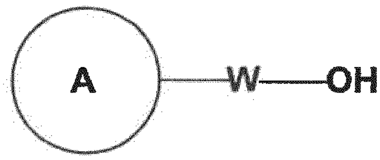 [I]

,

wherein the A moiety and W each has the same meaning as defined in Claim 1, or a pharmaceutically acceptable salt thereof, wherein the composition has a high penetration property into lens.

7. The agent/composition according to any one of Claims 1 to 6, wherein the A moiety is

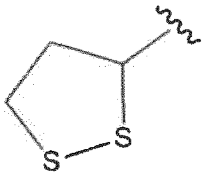 , 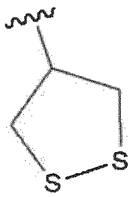 , 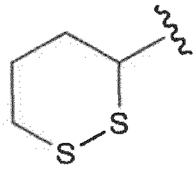 , 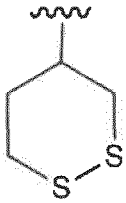 , 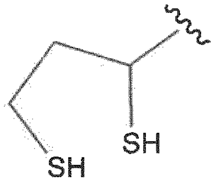 ,

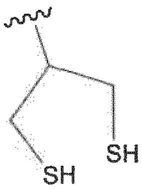 , 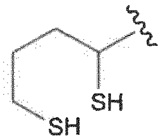 , or, 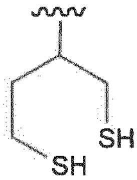 ,

wherein the A moiety may be optionally substituted with the same or different one to three $R^1$.

8. The agent/composition according to any one of Claims 1 to 7, wherein the A moiety is

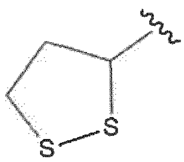 or 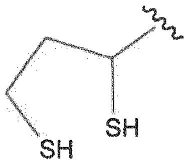 ,

wherein the A moiety may be optionally substituted with the same or different one to three $R^1$.

9. The agent/composition according to any one of Claims 1 to 8, wherein the A moiety is

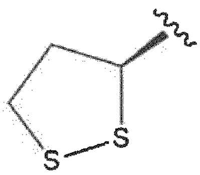 or 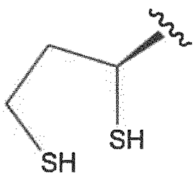 ,

wherein the A moiety may be optionally substituted with the same or different one to three $R^1$.

10. The agent/composition according to any one of Claims 1 to 9, wherein the A moiety is

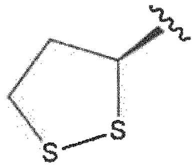

or

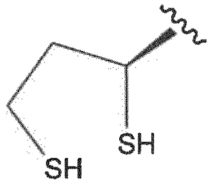

.

**11.** The agent/composition according to any one of Claims 1 to 10, wherein W is alkylene which is $-(CH_2)_{1-10}-$, wherein the alkylene may be optionally substituted with at least one substituent independently selected from the group consisting of -OH, halo, $C_{1-3}$ alkyl, and $C_{1-3}$ alkoxy.

**12.** The agent/composition according to any one of Claims 1 to 11, wherein W is alkylene which is $-(CH_2)_{1-10}-$.

**13.** The agent/composition according to any one of Claims 1 to 10, wherein W is

$-(CH_2)_{1-5}-O-(CH_2)_{2-4}-$,

$-(CH_2)_{1-2}-O-(CH_2)_{2-3}-O-(CH_2)_{2-3}-$,

$-(CH_2)_{1-5}-S-(CH_2)_{2-4}-$,

$-(CH_2)_{1-2}-S-(CH_2)_{2-3}-S-(CH_2)_{2-3}-$,

$-(CH_2)_{1-5}-NH-(CH_2)_{2-4}-$,

or

$-(CH_2)_{1-2}-NH-(CH_2)_{2-3}-NH-(CH_2)_{2-3}-$,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety, and the W may be optionally substituted with at least one substituent independently selected from the group consisting of -OH, halo, $C_{1-3}$ alkyl, and $C_{1-3}$ alkoxy.

**14.** The agent/composition according to any one of Claims 1 to 10 and 13, wherein W is

$-(CH_2)_{1-5}-O-(CH_2)_{2-4}-$,

$-(CH_2)_{1-2}-O-(CH_2)_{2-3}-O-(CH_2)_{2-3}-$,

$-(CH_2)_{1-5}-S-(CH_2)_{2-4}-$,

$-(CH_2)_{1-2}-S-(CH_2)_{2-3}-S-(CH_2)_{2-3}-$,

$-(CH_2)_{1-5}-NH-(CH_2)_{2-4}-$,

or

$-(CH_2)_{1-2}-NH-(CH_2)_{2-3}-NH-(CH_2)_{2-3}-$,

wherein the leftmost bond is attached to the A moiety, and the rightmost bond is attached to the oxygen atom of the hydroxy group, or the leftmost bond is attached to the oxygen atom of the hydroxy group, and the rightmost bond is attached to the A moiety.

**15.** The agent/composition according to any one of Claims 1 to 14, comprising, as an active ingredient, a compound selected from the group consisting of:

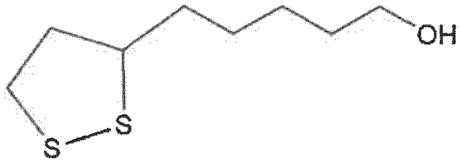
,
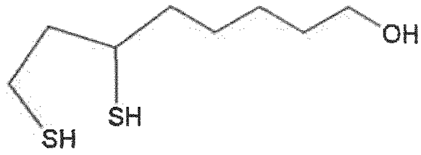
,

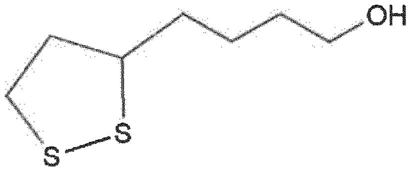
,
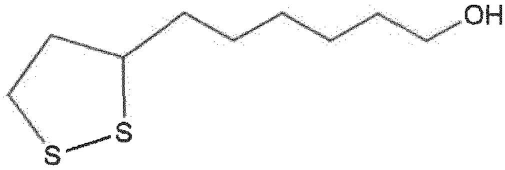
,

and

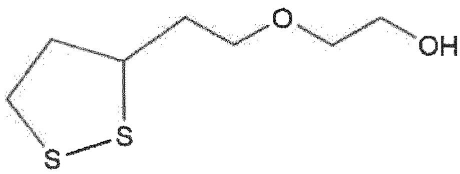
,

or a pharmaceutically acceptable salt thereof.

**16.** The agent/composition according to any one of Claims 1 to 15, comprising, as an active ingredient, a compound selected from the group consisting of:

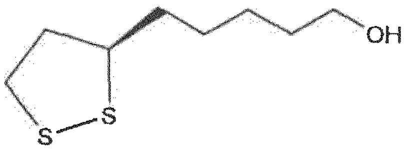
,
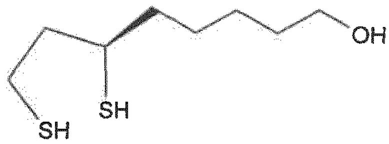
,

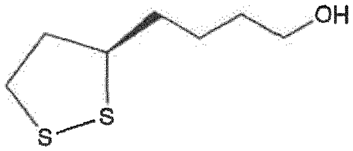
,
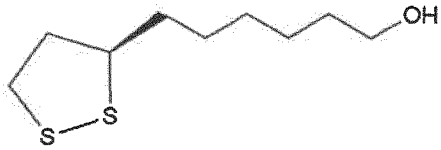
,

and

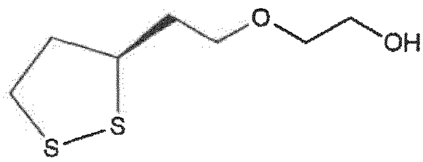
,

or a pharmaceutically acceptable salt thereof.

**17.** The agent/composition according to any one of Claims 1 to 12 and 15 to 16, comprising, as an active ingredient, a compound:

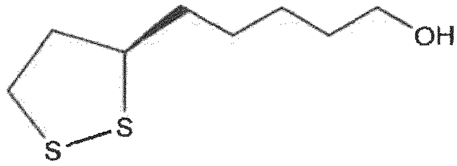

,

or a pharmaceutically acceptable salt thereof.

**18.** The agent/composition according to any one of Claims 1 to 12, and 15 to 16, comprising, as an active ingredient, a compound:

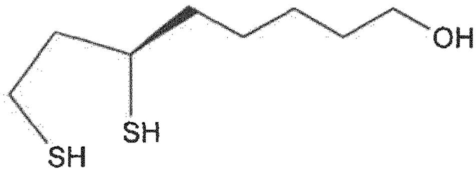

,

or a pharmaceutically acceptable salt thereof.

**19.** The agent/composition according to any one of Claims 1 to 18, wherein the agent/composition is for ophthalmic administration.

**20.** The agent/composition according to any one of Claims 1 to 19, wherein the agent/composition is an eye drop or an eye ointment.

**21.** The agent/composition according to any one of Claims 1 to 20, wherein the amount of the active ingredient comprised in the agent/composition is 0.00001 to 10% (w/v).

**22.** Use of a compound of Formula [I]

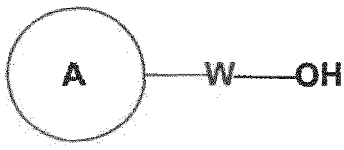

[I]

wherein the A moiety and W each has the same meaning as defined in Claim 1
or a pharmaceutically acceptable salt thereof, in the manufacture of an agent/composition for treating or preventing presbyopia, an eye disease accompanied by a decrease in lens elasticity, or an eye disease accompanied by a decrease in accommodative function of the eye.

**23.** A compound of Formula [I]

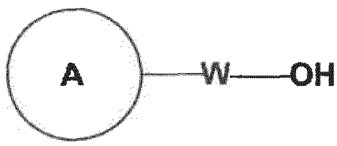

[I]

wherein the A moiety and W each has the same meaning as defined in Claim 1
or a pharmaceutically acceptable salt thereof, for use in the treatment or prevention of presbyopia, an eye disease accompanied by a decrease in lens elasticity, or an eye disease accompanied by a decrease in accommodative function of the eye.

**24.** A method for treating or preventing presbyopia, an eye disease accompanied by a decrease in lens elasticity, or an eye disease accompanied by a decrease in accommodative function of the eye, comprising administering to a

subject in need thereof an effective amount of a compound of Formula [I]

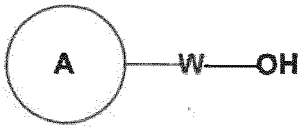 [I]

wherein the A moiety and W each has the same meaning as defined in Claim 1 or a pharmaceutically acceptable salt thereof.

**25.** A compound of Formula [I]

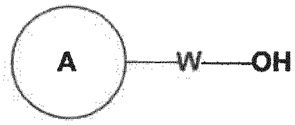 [I]

wherein the A moiety and W each has the same meaning as defined in Claim 1, excluding the following compounds:

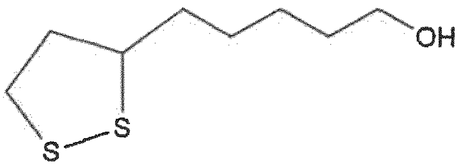 , and 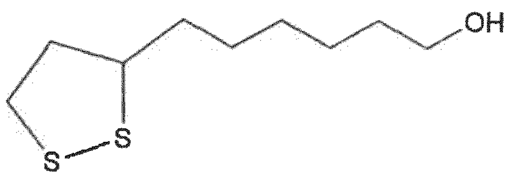 ,

or a pharmaceutically acceptable salt thereof.

**26.** A compound selected from the group consisting of:

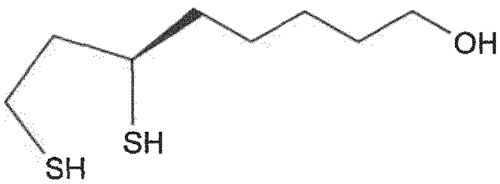 , 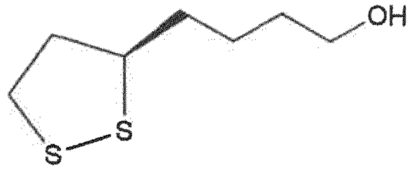 ,

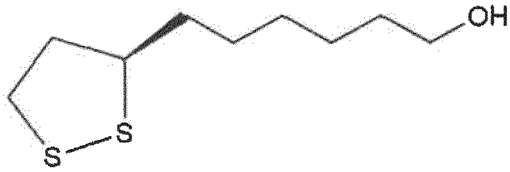 , 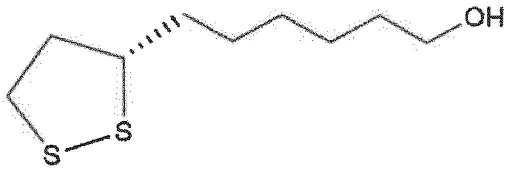 ,

and

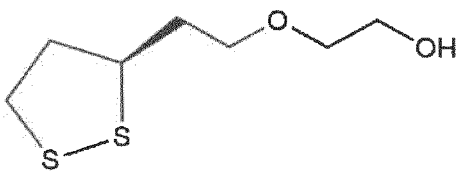 ,

or a pharmaceutically acceptable salt thereof.

**INTERNATIONAL SEARCH REPORT**

International application No.
PCT/JP2021/019808

A. CLASSIFICATION OF SUBJECT MATTER
A61K 31/095(2006.01)i; A61K 31/385(2006.01)i; A61P 27/02(2006.01)i; A61P 27/10(2006.01)i; C07C 323/12(2006.01)i; C07D 339/04(2006.01)i
FI: A61K31/095; A61P27/10; C07C323/12; C07D339/04 CSP; A61K31/385; A61P27/02

According to International Patent Classification (IPC) or to both national classification and IPC

B. FIELDS SEARCHED

Minimum documentation searched (classification system followed by classification symbols)
A61K31/095; A61K31/385; A61P27/02; A61P27/10; C07C323/12; C07D339/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X | JP 2004-331514 A (MITSUI CHEMICALS, INC.) 25 November 2004 (2004-11-25) claims, paragraph [0012], examples 4, 8, 12 | 6-9, 11, 12, 21, 23, 25 |
| X | CN 104231119 A (CHINA PETROLEUM & CHEMICAL CORPORATION) 24 December 2014 (2014-12-24) claims, paragraph [0010] | 6-12, 23, 25 |
| X | US 2017/0217944 A1 (YEOMYUNG BIOCHEM CO., LTD.) 03 August 2017 (2017-08-03) claims, paragraphs [0052]-[0054], formula 3 | 6, 11, 12, 23, 25 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 July 2021 (20.07.2021) | 10 August 2021 (10.08.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2021/019808

C (Continuation). DOCUMENTS CONSIDERED TO BE RELEVANT

| Category* | Citation of document, with indication, where appropriate, of the relevant passages | Relevant to claim No. |
|---|---|---|
| X | ALGAR, W. Russ et al., "Multidentate Surface Ligand Exchange for the Immobilization of CdSe/ZnS Quantum Dots and Surface Quantum Dot-Oligonucleotide Conjugates", Langmuir, 2008, vol. 24, no. 10, pp. 5514-5520, in particular, page 5515, right column, last paragraph | 6-12, 15, 16, 18, 23, 25, 26 |
| X | HIRAI, N. et al., "Allelochemicals of the tropical weed Sphenoclea zeylanica", Phytochemistry, 2000, vol. 55, no. 2, pp. 131-140, in particular, compound 23, fig. 2, page 138, left column, paragraph [0003] | 6-12, 23, 25 |
| X | SCHMIDT, Ulrich et al., "Lipoic acids. IV. Synthesis of lipoic acid antagonists". II, Justus Liebigs Annalen der Chemie, 1963, vol. 670, pp. 157-168, in particular, page 168, lines 3-12 | 6-11, 23, 25 |
| X | US 2811531 A (MERCK & CO., INC.) 29 October 1957 (1957-10-29) claims, column 1, example 1 | 6-12, 15-17, 23 |
| X | "DATABASE REGISTRY [Online]", Chemical Abstracts Service, Columbus, Ohio, USA, [retrieved on 20 July 2021], Retrieved from STN, REGISTRY No. 2354641-80-6, 10 July 2019, in particular, section "RN, CN, SR, chemical structural formula" | 6-12, 15, 16, 23, 26 |
| A | US 2010/0317725 A1 (ENCORE HEALTH LLC) 16 December 2010 (2010-12-16) claims, each example | 1-26 |
| A | US 2010/0317608 A1 (ENCORE HEALTH LLC) 16 December 2010 (2010-12-16) claims, each example | 1-26 |
| A | JP 2009-501727 A (CHAKSHU RESEARCH INC.) 22 January 2009 (2009-01-22) claims, paragraph [0023], table 1, each example | 1-26 |
| A | JP 2000-169371 A (SANKYO CO., LTD.) 20 June 2000 (2000-06-20) claims, paragraph [0135], formulation examples 5, 6, each example | 1-26 |

Form PCT/ISA/210 (continuation of second sheet) (January 2015)

**INTERNATIONAL SEARCH REPORT**
Information on patent family members

International application No.
PCT/JP2021/019808

| Patent Documents referred in the Report | Publication Date | Patent Family | Publication Date |
|---|---|---|---|
| JP 2004-331514 A | 25 Nov. 2004 | (Family: none) | |
| CN 104231119 A | 24 Dec. 2014 | (Family: none) | |
| US 2017/0217944 A1 | 03 Aug. 2017 | KR 10-1601685 B1 | |
| US 2811531 A | 29 Oct. 1957 | (Family: none) | |
| US 2010/0317725 A1 | 16 Dec. 2010 | WO 2010/147962 A1<br>EP 2442645 A1 | |
| US 2010/0317608 A1 | 16 Dec. 2010 | WO 2009/111635 A2<br>WO 2010/147957 A2<br>KR 10-2007-0060165 A<br>CN 101083999 A<br>EP 2821405 A1 | |
| JP 2009-501727 A | 22 Jan. 2009 | US 2006/0166879 A1<br>claims, paragraph [0025], table 1, each example<br>WO 2007/011874 A2<br>EP 1904108 A2<br>CN 101262886 A | |
| JP 2000-169371 A | 20 Jun. 2000 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010147957 A


### Non-patent literature cited in the description


- Atarashii ganka. *A New Ophthalmology,* 2011, vol. 28 (7), 985-988
- *InvestOphthalmol Vis Sci,* 2016, vol. 57, 2851-2863